(19) Europäisches Patentamt
European Patent Office
Office européen des brevets

(11) EP 2 298 936 A1

(12) **EUROPEAN PATENT APPLICATION**
published in accordance with Art. 153(4) EPC

(43) Date of publication:
**23.03.2011 Bulletin 2011/12**

(21) Application number: **09772596.4**

(22) Date of filing: **01.07.2009**

(51) Int Cl.:
***C12Q 1/68*** *(2006.01)* ***C12N 15/12*** *(2006.01)*
***G01N 33/53*** *(2006.01)*

(86) International application number:
**PCT/ES2009/070268**

(87) International publication number:
**WO 2010/000907 (07.01.2010 Gazette 2010/01)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **02.07.2008 ES 200802024**

(71) Applicant: **CENTRO DE INVESTIGACIONES ENERGETICAS MEDIOAMBIENTALES Y TECNOLOGICAS (C.I.E.M.A.T.)**
**E-28040 Madrid (ES)**

(72) Inventors:
- **GARCÍA ESCUDERO, Ramón**
  **E-28040 Madrid (ES)**
- **PARAMIO GONZÁLEZ, Jesús María**
  **E-28040 Madrid (ES)**
- **MARTÍNEZ CRUZ, Ana Belén**
  **E-28040 Madrid (ES)**
- **SANTOS LAFUENTE, Mirentxu**
  **E-28040 Madrid (ES)**

(74) Representative: **ABG Patentes, S.L.**
**Avenida de Burgos 16D**
**Edificio Euromor**
**28036 Madrid (ES)**

# (54) GENOMIC FINGERPRINT OF MAMMARY CANCER

(57) The present invention relates to *in vitro* methods for determining the prognosis of a subject diagnosed with breast cancer developing metastasis or for selecting suitable treatment for said subject. Particularly, the invention relates to a signature of genes the expression of which is correlated with the prognosis of a subject who has been diagnosed with breast cancer.

EP 2 298 936 A1

**Description**

Field of the Invention

The present invention relates to *in vitro* methods for determining the prognosis of a subject diagnosed with breast cancer for developing metastasis or for selecting suitable treatment for said subject.

Background of the Invention

Breast cancer is the second most common type of cancer worldwide (10.4%, after lung cancer) and the fifth most common cause of cancer-induced death (after lung cancer, stomach cancer, liver cancer, and colon cancer). Breast cancer is the most common cause of cancer-induced death among women worldwide. In 2005, breast cancer caused 502,000 deaths all over the world (7% of cancer-induced deaths; almost 1% of all deaths). The number of cases worldwide has significantly increased since the 1970s, a phenomenon which can partially be attributed to modern Western lifestyles. Women in North America have the highest incidence of breast cancer in the world.

Since the breast is made up of identical tissues in men and women, breast cancer also occurs in men. The incidence of breast cancer in men is approximately 100 times less than in women, but it is considered that men with breast cancer statistically have the same survival rates as women.

Breast cancer is staged according to the TNM system. The prognosis is closely related to the results of the staging, and the staging is also used to assign patients to treatments both in clinical trials and in medical practice. The information for staging is as follows:

- TX: The primary tumor cannot be evaluated. T0: There is no evidence of tumor. Tis: Carcinoma in situ, no invasion. T1: The tumor is 2 cm or less across. T2: The tumor is more than 2 cm but less than 5 cm across. T3: The tumor is more than 5 cm across. T4: Tumor of any size growing into the chest wall or skin, or inflammatory breast cancer.
- NX: The nearby lymph nodes cannot be evaluated. N0: The cancer has not spread to regional lymph nodes. N1: The cancer has spread to 1 to 3 underarm lymph nodes or to an internal mammary lymph node. N2: The cancer has spread to 4 to 9 underarm lymph nodes or to multiple internal mammary lymph nodes. N3: Any of the following:

    ■ The cancer has spread to 10 or more underarm lymph nodes, or the cancer has spread to the lymph nodes under the clavicle, or the cancer has spread to the lymph nodes above the clavicle or the cancer affects underarm lymph nodes and has spread to internal mammary lymph nodes, or the cancer affects 4 or more underarm lymph nodes, and small amounts of cancer are found in the internal mammary lymph nodes or in sentinel lymph node biopsy.

- MX: The presence of distant spread (metastasis) cannot be evaluated. M0: No distant spread is found. M1: Spread to distant organs is present, these organs not including the lymph node above the clavicle.

The principal pillar of breast cancer treatment is surgery when the tumor is localized, with possible adjuvant hormone therapy (with tamoxifen or an aromatase inhibitor), chemotherapy, and/or radiotherapy. Current recommendations for treatment after surgery (adjuvant therapy) follow a pattern. This pattern is subject to change, because every two years, a world conference is held in St. Gallen, Switzerland to discuss the actual results of multicenter studies conducted worldwide. Likewise, ·said pattern is also reviewed according to the consensual criterion of the National Institute of Health (NIH). Based on these criteria, over 85-90% of the patients who do not present metastasis in lymph nodes would be candidates for receiving adjuvant systemic therapy.

Today no set of predictors of a satisfactory prognosis based solely on clinical information has been identified. Over the last 30 years oncologists have focused on optimizing the outcome of the cancer patients and it is only now that the new available technologies allow investigating polymorphisms, expression levels of genes and gene mutations for the purpose of predicting the impact of a determined therapy on different groups of cancer patients in order to design customized chemotherapies. PCR assays such as Oncotype DX or microarray assays such as MammaPrint can predict the risk of breast cancer relapse based on gene expression. In February 2007, the MammaPrint assay became the first breast cancer indicator to receive official authorization from the Food and Drug Administration.

Document WO02103320 describes a method for predicting the prognosis of breast cancer patients by means of analyzing the expression of a group of genetic markers, particularly 70 genes (Table 6, page 89). D1 also describes a microarray for determining the prognosis of breast cancer from a sample from a patient comprising probes for detecting the gene expression of said genes.

In addition, document WO2005/083429 describes a method for selecting a genetic marker signature for the prognosis of breast cancer. Said document also describes a method for determining the prognosis of patients with breast cancer by means of analyzing the expression of a group of genes selected from said method. Specifically, said document relates to the use of genetic markers for predicting the prognosis of breast cancer from a characteristic signature consisting of 76 genetic markers. Said document also describes a kit, such as a microarray, for determining the prognosis of breast cancer from a sample from a patient comprising probes for detecting the gene expression of said 76 genes.

Therefore, there is a need to develop new methods which allow identifying the most relevant genes involved in metastasis such that more reliable signatures can be obtained and these signatures can be based on a smaller number of genes. Said signatures will allow predicting the prognosis of a patient suffering breast cancer more efficiently than the methods described in the state of the art. The identification of new prognosis factors will serve as a guideline in selecting the most suitable treatments.

## Summary of the Invention

In a first aspect, the present invention relates to an *in vitro* method for determining the prognosis of a subject diagnosed with breast cancer or for selecting the treatment of a subject diagnosed with breast cancer which comprises determining the expression levels of the genes identified in Table 1 and in Table 2 in a tumor tissue sample from said subject, wherein an increase of the expression of the genes identified in Table 1 and a decrease of the expression of the genes identified in Table 2 with respect to a reference value is indicative of a worse prognosis or of said subject having to be treated with chemotherapy.

In a second aspect, the invention relates to a reagent capable of detecting the expression levels of the genes of Tables 1 and 2.

In another aspect, the invention relates to a kit comprising at least one reagent according to the invention.

In another aspect, the invention relates to the use of a kit according to the invention for the prognosis of patients diagnosed with breast cancer or for selecting the treatment of a subject diagnosed with breast cancer.

The invention also relates in another aspect to a method for selecting genetic markers for predicting the tendency to develop metastasis of a primary tumor comprising the following steps:

i) determining the genes the expression of which is altered with respect to a reference value in a tumor sample from a genetically modified non-human animal showing a tendency to develop tumors spontaneously;
ii) identifying the homologous genes in humans corresponding to the genes identified in step i); and
iii) selecting those genes identified in step ii) the expression of which in primary tumor samples from patients who develop metastasis from said primary tumor is altered with respect to the expression of said genes in primary tumors of patients who do not develop metastasis.

## Brief Description of the Drawings

Figure 1 shows Receiver Operating Curves (ROC) of predicting the genetic signature of the invention, in the complete Loi dataset (n=191), or in the Loi dataset excluding grade 3 ER- samples (n=142). Panels A and C show the prediction of distant metastasis (DM) at 5 years, and panel E shows the prediction of overall survival at 5 years. Panels B and D show the prediction of distant metastasis (DM) at 10 years, and panel E shows the prediction of overall survival at 10 years. The RV of the point of maximum Specificity (40.1%) and Sensitivity (100%) of panel A was chosen as the threshold for separating into samples of good or poor prognosis (RV=-39.2) (circle).

Figure 2 shows survival curves or prognostic prediction of patients not treated with tamoxifen, and N- within the Loi dataset (86 tumors, diameter ≤ 5 cm).

Figure 3 shows survival curves or prognostic prediction of patients treated with tamoxifen, and N+ within the Loi dataset (108 tumors, diameter ≤ 5 cm).

Figure 4 shows the genetic signature of the invention in the Desmedt dataset. The left panel shows an expression map of the genes the sequences of which hybridize with the probes of the signature of the invention in the Desmedt tumors, excluding the grade 3 ER- (142 samples). The columns represent genes, and the rows represent samples. The genes are arranged from left to right according to decreasing values of the Wald statistic value in accordance with Table 2. The expression values are shown as $\log_2$ (mean=0, standard deviation=1).

The right panel shows the prediction results using the signature of genes of the invention, or using the criteria of St. Gallen (SG), NIH, Nottingham Prognostic Index (NPI), Adjuvant Online (AOL), and the 76-gene genomic signature of Veridex. Samples in light gray indicate a good prognosis; samples in dark gray indicate a poor prognosis. The presence of 37 probes, the expression of which is higher in the poor prognosis samples, can be observed. The ER status (ER+ in black, ER- in white), as well as the existence of DM at 5 or 10 years (presence of metastasis in white, absence in black), are shown. The genes are represented both by the Affymetrix probe identifiers, and by the NCBI gene symbols.

Figure 5 shows the genetic signature of the invention in the Loi dataset. A) The left panel shows an expression map of the genes the sequences of which hybridize with the probes in the Loi tumors, which were N- and were not treated with tamoxifen, excluding grade 3 ER- (86 samples). The columns represent genes and the rows represent samples. The genes are arranged from left to right according to decreasing values of the Wald statistic value in accordance with Table 2. The expression values are shown as $log_2$ (mean=0, standard deviation=1).

The right panel shows the prediction results using the signature of genes of the invention, or the criteria of St. Gallen (SG) and NIH. Samples in light gray indicate a good prognosis; samples in dark gray indicate a poor prognosis. The presence of 37 probes, the expression of which is greater the poor prognosis samples, can be observed. The ER status (ER+ in black, ER- in white), as well as the existence of DM at 5 or 10 years (presence of metastasis in white, absence in black), are shown. The genes are represented both by the Affymetrix probe identifiers and by the NCBI gene symbols.

B) This panel is similar to panel A, but it is for the group of 108 patients of the Loi dataset who were N+ and received hormone therapy.

Figure 6 shows the ranking of the genes selected in the comparison between murine tumors p53- and p53-; pRb- and normal skin. The genes which are overexpressed in mouse tumors arranged according to the magnitude of the relative change in gene expression (from 80.3 up to 1.2 times, panel A), or arranged by the P value (panel C), computed according to the Student's t-Test. The black color in the left column indicates genes increased more than 2 times. The dark gray in the central column indicates genes which also met the differential expression criteria of the SAM test. The position within the range of the equivalent mouse genes of the signature of genes of the invention in humans is indicated in the right column. Genes the name of which appears in dark gray are overexpressed in malignant human breast tumors; genes in light gray are decreased in malignant tumors

Genes having a reduced expression in the mouse tumors arranged according to the magnitude of the relative change in gene expression (from 375.8 up to 1.2 times, panel B), or arranged by the P value (panel D), computed according to the Student's t-Test. The black color of the left column indicates genes regulated negatively by more than 2 times. The dark gray in the column central indicates genes which also met the differential expression criteria of the SAM test. The position within the range of the equivalent mouse genes of the signature of genes in humans is indicated in the right column. Genes the name of which appears in dark gray are overexpressed in malignant human breast tumors; genes in light gray are decreased in malignant tumors.

Detailed Description of the Invention

The authors of the present invention have selected a signature of genes which hybridize with probes and the expression of which is correlated with the prognosis of a subject who has been diagnosed with breast cancer. Said signature can also be used for selecting the most suitable treatment for said subject diagnosed with breast cancer.

As hereinbefore mentioned, the criteria of St. Gallen and of the NIH classify patients as high risk or low risk based on several histological and clinical characteristics. The authors of the present invention have demonstrated that said prognostic signature of the invention assigns more patients to the low risk (or good prognosis) group than the traditional methods do. In fact, the inventors have shown that said clinical criteria mistakenly classify a clinically significant number of patients in the poor prognosis group, so in current clinical practice many patients are receiving chemotherapy unnecessarily.

Therefore, in a first aspect the invention relates to an *in vitro* method for determining the prognosis of a subject diagnosed with breast cancer or for selecting the treatment of a subject diagnosed with breast cancer which comprises determining the expression levels of the genes identified in Table 1 and in Table 2 in a tumor tissue sample from said subject, wherein an increase of the expression of the genes identified in Table 1 and a decrease of the expression of the genes identified in Table 2 with respect to a reference value is indicative of a worse prognosis or of said subject having to be treated with chemotherapy. In a particular embodiment of the invention, said tumor tissue sample is a primary tumor sample, particularly, said tumor is breast cancer. Thus, by way of illustration said tumor tissue sample can be a biopsy sample obtained, for example, by surgical resection.

In a particular embodiment of the method of the invention, said genes are the genes the nucleotide sequences of which hybridize with the probes identified in Table 1 and Table 2.

Table 1

| Gene symbol |
|---|
| TOP2A |
| TOMM70A |
| PLK1 |
| CCNB2 |
| UBEC2C |
| SPAG5 |
| CDC2 |
| MAD2L1 |
| BUB1B |
| TRIP13 |
| AURKA |
| KIF11 |
| BRCA1 |
| HMMR |
| CIAPIN1 |
| LRP8 |
| AURKB |
| CDKN3 |
| HSP90AA1 |
| NUSAP1 |
| ERO1L |
| MLF1IP |
| DCC1 |
| C21orf45 |
| PBK |
| ATAD5 |
| MCM10 |
| CDCA3 |
| RACGAP1 |

Table 2

| Gene symbol |
|---|
| ELOVL5 |
| PARP3 |
| CBX7 |

Table 3

| Symbol | SEQ ID NO |
|---|---|
| TOP2A | SEQ ID NO: 1 - SEQ ID NO: 11 |
| TOP2A-2 | SEQ ID NO: 12 - SEQ ID NO: 22 |
| TOMM70A | SEQ ID NO: 23 - SEQ ID NO: 33 |
| PLK1 | SEQ ID NO: 34 - SEQ ID NO: 44 |
| CCNB2 | SEQ ID NO: 45 - SEQ ID NO: 55 |
| UBEC2C | SEQ ID NO: 56 - SEQ ID NO: 66 |
| SPAG5 | SEQ ID NO: 67 - SEQ ID NO: 77 |
| CDC2 | SEQ ID NO: 78 - SEQ ID NO: 88 |
| CDC2-2 | SEQ ID NO: 89 - SEQ ID NO: 99 |
| MAD2L1 | SEQ ID NO: 100 - SEQ ID NO: 110 |
| BUB1B | SEQ ID NO: 111 - SEQ ID NO: 121 |
| TRIP13 | SEQ ID NO: 122 - SEQ ID NO: 132 |
| AURKA | SEQ ID NO: 133 - SEQ ID NO: 143 |
| KIF11 | SEQ ID NO: 144 - SEQ ID NO: 154 |
| BRCA1 | SEQ ID NO: 155 - SEQ ID NO: 165 |
| HMMR | SEQ ID NO: 166 - SEQ ID NO: 176 |
| CIAPIN1 | SEQ ID NO: 177 - SEQ ID NO: 187 |
| LRP8 | SEQ ID NO: 188 - SEQ ID NO: 198 |
| CIAPIN1-2 | SEQ ID NO: 199 - SEQ ID NO: 209 |
| AURKB | SEQ ID NO: 210 - SEQ ID NO: 220 |
| HMMR-2 | SEQ ID NO: 221 - SEQ ID NO: 231 |
| CDKN3 | SEQ ID NO: 232 - SEQ ID NO: 242 |
| HSP90AA1 | SEQ ID NO: 243 - SEQ ID NO: 253 |
| BRCA1-2 | SEQ ID NO: 254 - SEQ ID NO: 264 |
| HSP90AA1-2 | SEQ ID NO: 265 - SEQ ID NO: 275 |
| HSP90AA1-3 | SEQ ID NO: 276 - SEQ ID NO: 286 |
| HSP90AA1-4 | SEQ ID NO: 287 - SEQ ID NO: 297 |
| NUSAP1 | SEQ ID NO: 298 - SEQ ID NO: 308 |
| ERO1L | SEQ ID NO: 309 - SEQ ID NO: 319 |
| MLF1IP | SEQ ID NO: 320 - SEQ ID NO: 330 |
| DCC1 | SEQ ID NO: 331 - SEQ ID NO: 341 |
| C21orf45 | SEQ ID NO: 342 - SEQ ID NO: 352 |
| PBK | SEQ ID NO: 353 - SEQ ID NO: 363 |
| ATAD5 | SEQ ID NO: 364 - SEQ ID NO: 374 |
| MCM10 | SEQ ID NO: 375 - SEQ ID NO: 385 |
| CDCA3 | SEQ ID NO: 386 - SEQ ID NO: 396 |
| RACGAP1 | SEQ ID NO: 397 - SEQ ID NO: 407 |

Table 4

| Symbol | SEQ ID NO |
|---|---|
| ELOVL5 | SEQ ID NO: 408 - SEQ ID NO: 418 |
| PARP3 | SEQ ID NO: 419 - SEQ ID NO: 429 |
| CBX7 | SEQ ID NO: 430 - SEQ ID NO: 440 |

The quantification of the expression levels of the genes identified in Table 1 and in Table 2 can be performed from the RNA resulting from the transcription of said genes (mRNA) or, alternatively, from the complementary DNA (cDNA) of said genes. Therefore, in a particular embodiment the quantification of the expression levels of the genes identified in Table 1 and in Table 2 comprises the quantification of the messenger RNA (mRNA) of said genes, or a fragment of said mRNA, the complementary DNA (cDNA) of said genes, or a fragment of said cDNA, or mixtures thereof.

Additionally, the method of the invention can include performing an extraction step for the purpose of obtaining the total RNA, which can be performed by means of conventional techniques (Chomczynski et al., Anal. Biochem., 1987, 162:156; Chomczynski P., Biotechniques, 1993, 15:532).

Virtually any conventional method can be used within the invention for detecting and quantifying the levels of mRNA encoded by the genes the nucleotide sequences of which hybridize with the probes of Tables 1 and 2 or of the corresponding cDNA thereof. By way of non-limiting illustration, the levels of mRNA encoded by said genes can be quantified by means of using conventional methods, for example, methods comprising the amplification of the mRNA and the quantification of the said mRNA amplification product, such as electrophoresis and staining, or alternatively, by means of Northern blot and using suitable probes, Northern blot and using probes specific for mRNA of the genes of interest or of the corresponding cDNA thereof, mapping with the S1 nuclease, RT-PCR, hybridization, microarrays, etc. Similarly, the levels of the cDNA corresponding to said mRNA encoded by the genes of Tables 1 and 2 can also be quantified by means of using conventional techniques; in this case, the method of the invention includes a step of synthesizing the corresponding cDNA by means of reverse transcription (RT) of the corresponding mRNA followed by amplification and quantification of the said cDNA amplification product. Conventional methods of quantifying expression levels can be found, for example, in Sambrook et al., 2001 "Molecular cloning: a Laboratory Manual", 3rd ed., Cold Spring Harbor Laboratory Press, N.Y., Vol. 1-3.

In a particular embodiment of the invention, the quantification of the expression levels of the genes identified in Tables 1 and 2 is performed by means of a quantitative multiplex polymerase chain reaction (PCR) or a DNA or RNA array.

In another particular embodiment of the invention, the determination of the expression levels of the genes identified in Table 1 and Table 2 is performed by means of a DNA array comprising the probes identified in Tables 3 and 4. In a more particular embodiment of the invention, said array comprises at least one set of 11 probes for determining the expression levels of each of the genes of Tables 1 and 2. Thus, for determining the expression levels of each of said genes, a mean of the signal of said 11 probes used for detecting the expression of said gene is calculated.

Thus, said method comprises determining the expression levels of said genes of Tables 1 and 2 with respect to a reference value. In a particular embodiment of the invention, said reference value is the gene expression value of said genes of Tables 1 and 2 in a primary tumor sample from patients who do not develop metastasis. Preferably, it will be considered that the genes present increased expression when the expression ratio of a gene is at least 1.5 times with respect to a reference value, preferably greater than 2 times, more preferably greater than 3, 4, 5 and 10 times. Likewise, in a particular embodiment of the invention, it will be considered that the genes present decreased expression with respect to a reference value when the expression ratio of a gene is at least 1.5 times less than the reference value.

In a particular embodiment of the invention, the method for determining the better or worse prognosis of a subject who has been diagnosed with breast cancer comprises performing a proportional hazards regression analysis depending on said expression values of the genes identified in Tables 1 and 2. According to the data shown in Example 2, the authors have demonstrated that in this manner, said prognosis is performed with an effectiveness which, according to the ROC curves, would have a sensitivity of 100% as well as maximum specificity. Therefore, in a particular embodiment of the invention, the determination of said prognosis comprises a proportional hazards regression analysis of said prognosis depending on the expression levels of the genes identified in Table 1 and in Table 2.

As is described in Example 2 enclosed in the present description, the inventors have used a Cox-type proportional hazards regression analysis for determining the prognosis of a subject diagnosed with breast cancer. Said Cox analysis assigns a regression coefficient for each gene, such that the gene the expression of which is directly correlated with the prognostic variable, for example with the onset of metastasis, is > 0, and if its expression is inversely related to said variable, it is < 0. Therefore, in a particular embodiment of the invention, said proportional hazards regression analysis is a Cox-type analysis. In a more particular embodiment, distant metastasis is established in said Cox-type analysis as a prognostic variable. In a preferred embodiment, said distant metastasis is distant metastasis at 5 or 10 years.

The inventors have demonstrated that by means of the method of the invention it is possible to determine the prognosis of a patient with high sensitivity and specificity. Thus, from the gene expression values for the genes of Tables 1 and 2 as has been hereinbefore described, and from the value of the Wald statistic of the proportional hazards regression analysis, the inventors have demonstrated that it is possible to determine said prognosis by applying the following formula:

$$\sum_{i=1}^{40} s_i \cdot x_i + 39.2$$

wherein $x_i$ is the value of the expression level in log2 of each of said genes identified in Tables 1 and 2; and $S_i$ is the value of the Wald statistic of the Cox-type regression analysis for each of said genes identified in Tables 1 and 2, wherein if the value obtained is greater than zero, then it is indicative of said patient presenting a worse prognosis or of said patient having to be treated with chemotherapy, and wherein if said value is less than zero, it is indicative of said patient presenting a good prognosis or of said patient not having to be treated with chemotherapy.

The value of the Wald statistic is a value commonly used by the person skilled in the art to known whether or not the variables which are introduced in the statistical analysis are relevant. Said value can be calculated as described in Wald A. (1943) (Transactions of the American Mathematical Society. 1943; 54:426-482) and Silvey (1959) (Silvey SD. Annals of Mathematical Statistics. 1959; 30:389-407).

In addition, besides quantifying the expression levels of the genes identified in Tables 1 and 2, the expression level of the proteins encoded by said genes can also be quantified for putting the invention into practice. Thus, in a particular embodiment the quantification of the levels of the proteins encoded by the genes identified in Tables 1 and 2 comprises the quantification of said proteins or variables thereof.

As it is used herein, the term "protein" relates to a molecular chain of amino acids attached by covalent or noncovalent bonds. The term further includes all the physiologically relevant forms of post-translational chemical modifications, for example, glycosylation, phosphorylation or acetylation, etc.

In the present invention "variant" is understood as a protein the amino acid sequence of which is substantially homologous to the amino acid sequence of a specific protein. An amino acid sequence is substantially homologous to a determined amino acid sequence when it presents at least a 70% degree of identity, advantageously at least 75%, typically at least 80%, preferably at least 85%, more preferably at least 90%, still more preferably at least 95%, 97%, 98% or 99%, with respect to said determined amino acid sequence. The degree of identity between two amino acid sequences can be determined by conventional methods, for example, by means of standard sequence alignment algorithms known in the state of the art, such as BLAST [Altschul S.F. et al. Basic local alignment search tool. J Mol Biol. 1990 Oct 5; 215(3):403-10], for example.

The person skilled in the art understands that the mutations in the nucleotide sequence of the genes which give rise to conservative substitutions of amino acids in noncritical positions for protein functionality are mutations with a neutral evolution which do not affect its overall structure or its functionality. Said variants fall within the scope of the present invention.

Therefore, as it is used herein, the term "variant" also includes any fragment of one of the proteins described in the present invention. The term "fragment" relates to a peptide comprising a portion of a protein.

The expression level of the proteins encoded by the genes identified in Tables 1 and 2 can be quantified by means of any conventional method which allows detecting and quantifying said proteins in a sample from a subject. By way of non-limiting illustration, the levels of said proteins can be quantified, for example, by means of using antibodies with the capacity to bind to said proteins (or to fragments thereof containing an antigenic determinant) and the subsequent quantification of the formed complexes. The antibodies which are used in these assays can be labeled or not. Illustrative examples of markers which can be used include radioactive isotopes, enzymes, flourophores, chemiluminescent reagents, enzyme substrates or cofactors, enzyme inhibitors, particles, dyes, etc. There is a wide range of known assays that can be used in the present invention which use non-labeled antibodies (primary antibody) and labeled antibodies (secondary antibody); these techniques include Western blot, ELISA (Enzyme-linked Immunosorbent Assay), RIA (Radioimmunoassay), competitive EIA (Competitive Enzyme Immunoassay), DAS-ELISA (Double Antibody Sandwich ELISA), immunocytochemical and immunohistochemical techniques, techniques based on using protein biochips or microarrays which include specific antibodies or assays based on colloidal precipitation in formats such as dipsticks. Other ways of detecting and quantifying said proteins include affinity chromatography techniques, ligand binding assays, etc.

In a particular embodiment, the quantification of the levels of protein encoded by the genes identified in Tables 1 and 2 is performed by means of Western blot, ELISA, immunohistochemistry or a protein array.

As hereinbefore mentioned, the *in vitro* method of the invention can be used for selecting the treatment of a subject diagnosed with breast cancer, wherein an increase of the expression of the genes identified in Table 1 and a decrease of the expression of the genes identified in Table 2 with respect to a reference value is indicative of said subject having to be treated with chemotherapy.

The suitable chemotherapy agents include but are not limited to alkylating agents such as, for example, cyclophosphamide, carmustine, daunorubicin, mechlorethamine, chlorambucil, nimustine, melphalan and the like; anthracylines, such as, for example, daunorubicin, doxorubicin, epirubicin, idarubicin, mitoxantrone, valrubicin and the like; taxane compounds, such as, for example, paclitaxel, docetaxel and the like; topoisomerase inhibitors such as, for example, etoposide, teniposide, irinotecan, tuliposide and the like; nucleotide analogues such as, for example, azacitidine, azathioprine, capecitabine, cytarabine, doxifluridine, fluorouracil, gemcitabine, mercaptopurine, methotrexate, thioguanine ftorafur and the like; platinum-based agents such as, for example, carboplatin, cisplatin, oxaliplatin and the like; antineoplastic agents such as, for example, vincristine, leucovorin, lomustine, procarbazine and the like; hormone modulators such as, for example, tamoxifen, finasteride, 5-$\alpha$-reductase inhibitors and the like; vinca alkaloids such as, for example, vinblastine, vincristine, vindesine, vinorelbine and the like. The suitable chemotherapy agents are described in detail in the literature, such as in The Merck Index in CD-ROM, 13$^{th}$ edition.

In the present invention, "antitumor agent" is understood as that chemical, physical or biological agent or compound with antiproliferative, antioncogenic and/or carcinostatic properties which can be used to inhibit tumor growth, proliferation and/or development. Examples of antitumor agents which can be used in the present invention are (i) antimetabolites, such as antifolates and purine analogues; (ii) natural products, such as antitumor antibiotics and mitotic inhibitors; (iii) hormones and antagonist thereof, such as androgens and corticosteroids; and (iv) biological agents, such as viral vectors. A list of compounds which can be used as antitumor agents is described in patent application WO2005/112973.

In another aspect, the present invention relates to a reagent, hereinafter reagent of the invention, capable of detecting the expression levels of the genes of Tables 1 and 2.

In a particular embodiment, said reagent of the invention comprises

(i) a set of nucleic acids comprising the nucleotide sequences of the probes identified in Tables 3 and 4 or the products of their transcription, or

(ii) a set of antibodies, or a fragment thereof, capable of detecting an antigen, consisting of each antibody or fragment being capable of binding specifically to one of the proteins encoded by the genes identified in Tables 1 and 2.

In a particular embodiment of the invention, said nucleic acids are DNA, cDNA or RNA probes and/or primers. Said nucleic acids can be obtained by conventional techniques known by the person skilled in the art from the nucleotide sequences of the genes of Tables 1 and 2. Said probes and/or primers can generally be obtained from companies by chemical synthesis. In addition, said sequences of said genes are well described in the literature and are therefore known.

In another aspect, the present invention relates to a kit comprising at least one reagent according to the invention. In a particular embodiment of the invention, said kit is a DNA or RNA array comprising a set of nucleic acids, wherein said set of nucleic acids comprises the nucleotide sequences of the probes of Tables 3 and 4, or a fragment thereof, or the products of their transcription. In a more particular embodiment, said kit further comprises a nucleic acid molecule of one or several constitutive expression genes.

In the present invention "genes which are expressed constitutively" or "constitutive expression genes" is understood as those genes that are always active or which are constantly transcribed. Examples of genes which are expressed constitutively are 2-myoglobin, ubiquitin, 18S ribosomal protein, cyclophilin A, transferrin receptor, actin, GAPDH, tyrosine 3-monooxygenase/tryptophan 5-monooxygenase activation protein (YWHAZ), ubiquitin, beta-actin and $\beta$-2-microglobulin.

In another particular embodiment of the invention, the kit of the invention comprises a set of antibodies, wherein said set of antibodies consists of antibodies or fragments thereof capable of binding specifically with the proteins encoded by the genes identified in Tables 1 and 2 or any variant of said proteins. In a particular embodiment, said kit further comprises antibodies or fragments thereof capable of binding specifically with the proteins encoded by one or several constitutive expression genes.

The term genetic signature or signature of genes of the invention as it is herein used relates to the genes identified in Tables 1 and 2. According to the data shown by the inventors (see Example 4), the signature of genes or genetic signature of the invention assigns more patients to the low risk (or good prognosis) group than traditional methods do. Thus, the inventors have demonstrated that the patients classified as poor prognosis patients according to the method of the present invention tend to have a greater proportion of metastasis than the poor prognosis patients according to the clinical criteria of St Gallen and NIH. Therefore, in another aspect, the invention relates to the use of a kit according to the invention for the prognosis of patients diagnosed with breast cancer or for selecting the treatment of a subject diagnosed with breast cancer.

In another aspect, the invention relates to a method for selecting genetic markers for predicting the tendency to develop metastasis of a primary tumor comprising the following steps:

i) determining the genes the expression of which is altered with respect to a reference value in a tumor sample from a genetically modified non-human animal showing a tendency to develop tumors spontaneously;
ii) identifying the homologous genes in humans corresponding to the genes identified in step i); and
iii) selecting those genes identified in step ii) the expression of which in primary tumor samples from patients who develop metastasis from said primary tumor is altered with respect to the expression of said genes in primary tumors of patients who do not develop metastasis.

For determining the genes the expression of which is altered according to step i), first a sample is obtained from said animal, preferably said sample is a tumor tissue sample. Example 1 of the present invention describes a particular embodiment of the method of the invention. Thus, in a particular embodiment, the total RNA is extracted from said tumor tissue sample of said animal and said RNA is analyzed to determine the genes the expression of which is altered in said tumor sample with respect to a reference value. In a particular embodiment, said reference value is the gene expression value in a non-tumor tissue sample from said animal. Said expression value can be obtained, for example, from the values resulting from the gene expression signal in a gene expression array of said non-human animal model as is explained in Example 1 of the present description. Thus, said values correspond with the values in the CEL (CEL format) type files according to the GCOS (GeneChip® Operating Software) software of Affymetrix.

In a particular embodiment of the method for selecting genetic markers of the invention, said non-human animal is an animal in which the gene expression of the Tp53 gene is inhibited. In another particular embodiment, the gene expression of the pRb gene is further inhibited in said animal. The Tp53 and Rb1 genes respectively encode tumor suppressors p53 and pRb. Said animal models, with a deficiency of the p53 and pRb genes (p53- and pRb-), spontaneously develop highly invasive epidermal carcinomas. Therefore, in a particular embodiment, said animal is a non-human animal which spontaneously develops epidermal carcinomas.

To carry out step ii) of the method for selecting genetic markers for predicting the tendency to develop metastasis of a primary tumor, the homologous genes in humans corresponding to the genes identified in step i) are identified. To that end, conventional techniques for mapping homologous genes known by the person skilled in the art are used. Particularly, the inventors have mapped the Affymetrix probe identifiers of the non-human animal with human gene symbols through the search for identifiers in U133plus 2.0 and U133A by means of using the AILUN (Array Information Library Universal Navigator) web utility (Chen R, et al. Nat Methods 2007;4(11):879).

In a final step of said method, those genes identified in step ii) the expression of which in primary tumor samples from patients who develop metastasis from said primary tumor is altered with respect to the expression of said genes in primary tumors of patients who do not develop metastasis are selected.

In a particular embodiment of the invention, step iii) is carried out by means of a proportional hazards regression analysis as hereinbefore mentioned. In a more particular embodiment, said regression analysis is a Cox-type analysis. In a preferred embodiment, said Cox-type method establishes distant metastasis as a prognostic variable. In an even more preferred embodiment of the invention, said distant metastasis is distant metastasis at 5 or 10 years.

The inventors have further applied the Wald test (Wald A. Transactions of the American Mathematical Society 1943;54:426-482; Silvey SD. Annals of Mathematical Statistics 1959; 30:389-407) to analyze the null hypothesis that the coefficient is 0 (not related to the prognostic variable), a Wald statistic value, the corresponding P value, and P value corrected by the FDR (false discovery rate) method as explained in Example 2 of the present description, being assigned to each gene. FDR control is a statistical method known by the person skilled in the art used in multiple hypothesis testing for correcting multiple comparisons.

In a particular embodiment of the invention, said primary tumors according to step iii) are breast cancer tumors.

In a particular embodiment, the determination of the expression of said genes according to step iii) comprises the quantification of the messenger RNA (mRNA) of said genes, or a fragment of said mRNA, the complementary DNA (cDNA), or a fragment of said cDNA, or mixtures thereof.

In a more particular embodiment, the quantification of the expression levels of the genes according to step iii) is performed by means of a quantitative multiplex polymerase chain reaction (PCR) or a DNA or RNA array.

In a particular embodiment, the quantification of the expression levels of the genes according to step iii) comprises the quantification of the levels of protein encoded by said genes. In a more particular embodiment, the quantification of the levels of protein is performed by means of Western blot, ELISA or a protein array.

The following Examples illustrate the invention and must not be interpreted as limiting of the scope thereof.

EXAMPLE 1

Analysis of mouse epidermal tumors

The animal models used in the present invention are K14Cre mice (they express Cre recombinase in the basal layer of stratified epithelia) crossed with mice with essential exons flanked by loxP sequences in the alleles of the Tp53 genes (p53-model), or in the Tp53 and Rb1 alleles simultaneously (p53-model; pRb- model) (Martinez-Cruz AB. et al. Cancer Res 2008; 68 (3) :683-692) . Tp53 and Rb1 respectively encode tumor suppressors p53 and pRb. Therefore they are gene deletion models in stratified epithelia. Both models spontaneously develop highly invasive poorly differentiated or undifferentiated type epidermal squamous cell carcinomas.

RNA from frozen epidermal carcinomas which occurred in mice deficient in p53 (p53-) (7 tumors) and deficient in p53 and pRb (p53-/pRb-) (8 tumors) was purified. RNA from normal skin preserved in RNAlater from adult animals (8 weeks old, 5 control samples) was obtained as controls. The integrity of the RNA populations was checked by means of using the Bioanalyzer system (Agilent). All the RNA samples met the quality criteria for microarray analysis (RIN number (RNA integrity number) above 6). Hybridization to the Affymetrix GeneChip, *Mouse Gene Expression* MOE430 2.0, was performed in the Genomic Department of the Cancer Research Center of Salamanca, using standard Affymetrix protocols. The expression values were extracted from the CEL files (resulting from the fluorescence scanning according to the Affymetrix GCOS software (GeneChip® Operating Software) by means of the RMA (Robust Multichip Average) method (Bolstad BM, et al. Bioinformatics 2003; 19(2):185-193; Irizarry RA, et al. Biostatistics 2003; 4(2):249-264). All the hybridizations met the quality criteria included in the RMAExpress computer program using RLE (Relative Log Expression) and NUSE (Normalized Unscaled Standard Error) graphics.

The analysis of differential gene expression of the mouse tumors compared with normal tissue were performed by means of the Student's t-Test (T-test) and SAM (Significant Analysis of Microarrays) (Tusher VG, et al. Proc Natl Acad Sci U S A 2001; 98(9):5116-5121) in the free Multiexperiment Viewer 4.0 software (MeV 4) (Saeed AI, et al. Biotechniques 2003; 34(2):374-378). The probes were selected if they met two criteria: i) T-test analysis with probability P value, corrected by the False Discovery Rate method (Benjamini Y, Hochberg Y. Journal of the Royal Statistical Society B 1995; 57:289-300) or FDR < $3\times10^{-7}$; and ii) SAM analysis with FDR < $1\times10^{-3}$. A total of 682 probes were selected as differentially expressed, 371 being overexpressed and 311 being negatively regulated in the tumors compared to normal tissue. The Affymetrix identifiers of the chip used (MOE430 2.0) were mapped to the homologous human gene symbol using the Ailun web utility (Chen R, et al. Nat Methods 2007; 4(11):879), which resulted in 427 human genes.

EXAMPLE 2

Two-step extraction of a breast cancer metastasis predictor based on a p53 signature.

2.1 Selection of genes with the p53 signature related to metastasis.

The raw data on the hybridization to microarrays of human primary breast tumors and their corresponding clinical data, obtained with the versions of Affymetrix *Human Gene Expression* U133A or U133Plus 2.0 GeneChips were downloaded from the Gene Expression Omnibus (GEO) web page database of the NCBI, with the identifiers GSE7390 (Desmedt C. et al. Clin Cancer Res 2007; 13(11):3207-3214) (study hereinafter referred to as Desmedt dataset) and GSE6532 (Loi S. et al. J Clin Oncol 2007;25(10):1239-1246) (study hereinafter referred to as Loi dataset). The CEL files were taken to extract the signal intensity values using the RMAExpress program. The RLE and NUSE graphics allowed identifying some tumor microarrays which did not meet the optimal normalization criteria with RMA. The corresponding CEL files were removed from subsequent analyses.

The Desmedt dataset was used as a training set which, after removing the low-quality arrays, contained 191 tumors with healthy lymph nodes (N-), including both samples which express estrogen receptor and samples which do not (ER+ or ER-, respectively) from patients who have not received adjuvant systemic therapy (Table 5).

Table 5: Clinical and pathological characteristics of the patients of the Desmedt dataset

| | Desmedt dataset (n=191) |
|---|---|
| Age (years) | |
| Mean | 46 years |
| <45 | 78 (41%) |
| 45-65 | 113 (59%) |

(continued)

| | Desmedt dataset (n=191) |
|---|---|
| Age (years) | |
| >65 | 0 (0%) |
| Size (cm) | |
| <1 | 8 (4%) |
| 1-2 | 90 (47%) |
| >2-5 | 93 (49%) |
| Degree of tumor differentiation | |
| Poor | 81 (42%) |
| Moderate | 80 (42%) |
| Good | 28 (15%) |
| Unknown | 2 (1%) |
| ER status | |
| Positive | 128 (67%) |
| Negative | 63 (33%) |
| Distant metastasis after 5 years | |
| Yes | 34 (18%) |
| No | 149 (78%) |
| Censored | 8 (4%) |

The data are numbers of patients, or percentages of patients. TM=tamoxifen

A Cox-type proportional hazards regression analysis was performed using distant metastasis (DM) at 5 years for the 707 U133A probes (and also present in U133Plus 2.0) corresponding to the 427 genes humans mapped from the analysis of differential expression of the mouse tumors, using the survival utility implemented on the GEPAS web page (www.gepas.org) (Vaquerizas JM. et al. Nucleic Acids Res 2005;33 (Web Server issue):W616-620).

Briefly, the Cox analysis assigns a Cox regression coefficient for each probe, such that a probe the expression of which is directly correlated with the occurrence of DM is > 0, and if its expression is inversely related to DM it is < 0. Furthermore the Wald test (Wald A. Transactions of the American Mathematical Society 1943;54:426-482; Silvey SD. Annals of Mathematical Statistics 1959; 30:389-407) is applied to analyze the null hypothesis that the coefficient is 0 (not related to DM), a Wald statistic value, the corresponding P value, and P value corrected by the FDR method, being assigned to each probe. The probes with Wald statistic values > 3 or <-3 were chosen for subsequent analyses. The purpose of these analyses is to check the DM prediction capacity DM at 5 and 10 years of human breast cancer.

2.2 Development of a mathematical model for the prediction of metastasis.

A formula was obtained to calculate a "risk value" (RV) of each tumor based on the described genes:

$$\text{Risk value(RV)} = \sum_{i=1}^{40} s_i \cdot x_i$$

wherein $s_i$ is the Wald statistic of the Cox-type regression analysis; and
$x_i$ is the expression value in log2 of the Affymetrix probe (mean=1; standard deviation=1)
Table 6 indicates the genes of the signature of the invention and the corresponding values of $s_i$

Said formula assigns a numerical value to each sample (RV) based on the sum of the products of the expression values of each gene and the values of the Wald statistic of each gene according to the Cox model hereinbefore explained (see Table 7).

The RV values of the 191 tumors of the Desmedt dataset were obtained according to the RV formula described above. Receiver Operating Curves (ROC) were computed for the RV using the variable of DM at 5 years as the censored dependent variable. As is shown in Figure 1, the RVs based on the signature of the genes identified in Table 1 and Table 2 allows predicting the absence of metastatic events at 5 years with a success rate of 100% (100% sensitivity) in a group of tumors (referred to hereinafter as the good prognosis group), and the presence of metastasis with a success rate of 40.1% (40.1% specificity) in the remaining tumors (referred to as the poor prognosis group).

A detailed analysis of the characteristics of the Desmedt tumors showed that those which were grade 3 ER- (46 samples) were not correctly predicted (data not shown). ROC curves of the remaining tumors showed that the signature of genes of the invention maintained sensitivity values of 100%, but it substantially improved the specificity up to 51.6% (Figure 1, Table 7).

Table 7: Parameters of the analysis of ROC curves

| | | Desmedt dataset (142 tumors) | |
|---|---|---|---|
| DM at 5 years | AUC | 0.846 | |
| | RV Threshold | -39.2 | |
| | | Sensitivity | Specificity |
| | | 100.0 | 51.6 |
| | | | |
| DM at 10 years | AUC | 0.819 | |
| | RV Threshold | -39.2 | |
| | | Sensitivity | Specificity |
| | | 96.0 | 53.0 |
| AUC=Area under the ROC curve | | | |

The results obtained demonstrate that the genetic signature of the invention could be used as an optimal predictor of DM at 5 years in human breast cancer.

Thus, from the formula hereinbefore described, the inventors have been able to determine whether a patient would belong to the good prognosis group or to the poor prognosis group. This method is based on the formula for calculating the risk value (RV) and on the ROC curve of DM at 5 years of the of Desmedt tumor group of 142 samples (Figure 1, panel C). According to the ROC curves, the predictor would have a sensitivity of 100 and maximum specificity (RV= - 39,2).

$$\text{Si} \sum_{i=1}^{40} s_i \cdot x_i + 39.2 > 0 \longrightarrow \text{Poor prognosis}$$

$$\text{Si} \sum_{i=1}^{40} s_i \cdot x_i + 39.2 < 0 \longrightarrow \text{Good prognosis}$$

EXAMPLE 3

Validation of the predictor in an external tumor group

The Loi tumor group or Loi dataset was used as an external tumor group for validation or testing dataset. The Loi dataset contains: i) tumors from patients treated or not treated with tamoxifen; ii) tumors from patients who had lymph node metastasis (N+) or not (N-) at the time of the operation; and iii) ER- or ER+ tumors. The Loi tumor group contains a more varied range of breast cancer samples than the Desmedt dataset (only N-, and not treated with tamoxifen). The Loi dataset originally has 327 samples analyzed using both the Affymetrix U133A GeneChip and the U133B GeneChip. It also contains 87 tumors analyzed with the U133Plus 2.0 chip. Since the genomic signature for prediction contains probes (Table 6) which are within the U133A GeneChip and not the U133B GeneChip, the analyses performed with the U133B GeneChip were discarded. However, the 87 samples analyzed with the U133Plus 2.0 chip will be processed because this chip contains all the U133A probes. After normalization with RMA, 400 tumors met the quality criteria hereinbefore explained (NUSE and RLE graphics).

The expression values were extracted in $\log_2$ scale for the genes of the signature of all the tumors. The risk value (RV) was calculated according to the formula hereinbefore described, the expression values of the new tumors and the Wald statistic values computed in the Cox analysis of the Desmedt dataset (Table 6) being used. The tumors for which there are no data on hormone treatment, tumor grade, presence or absence of ER, presence or absence of distant metastasis with follow-up over time, or the lymph node status, were discarded (94 samples). Out of the remaining 306 tumors, those tumors for which the genomic predictor did not work in the Desmedt dataset, i.e., grade 3 ER-tumors (19 samples), were eliminated. In the remaining 287 tumors, the precision of the genomic predictor was analyzed by groups of patients with similar characteristics.

3.1. The genetic signature of the invention is a genomic predictor of distant metastasis in breast cancer patients with healthy lymph nodes and who did not receive hormone therapy.

First the tumors with characteristics similar to those of the tumors of the Desmedt study, i.e., patients not treated with tamoxifen, N- nodes, and tumor diameter ≤ 5 cm, were analyzed but this analysis was independent of the age of the patient (86 samples, see Table 8 with clinical characteristics).

Table 8: Clinical and pathological characteristics of the patients of the Loi dataset

| | Loi Dataset | |
|---|---|---|
| | N- No TM (n=86) | N+ TM (n=108) |
| Age (years) | | |
| Mean | 52 years | 64 years |
| <45 | 19 (22%) | 1 (1%) |
| 45-65 | 65 (76%) | 62 (57%) |
| >65 | 2 (2%) | 45 (42%) |
| Size (cm) | | |
| <1 | 3 (4%) | 1 (1%) |
| 1-2 | 51 (59%) | 35 (32%) |
| >2-5 | 32 (37%) | 72 (67%) |
| Degree of tumor differentiation | | |
| Poor | 12 (14%) | 20 (19%) |
| Moderate | 47 (55%) | 65 (60%) |
| Good | 27 (31%) | 23 (21%) |
| Unknown | | |
| ER status | | |
| Positive | 69 (80%) | 107 (99%) |
| Negative | 17 (20%) | 1 (1%) |
| Distant metastasis after 5 years | | |
| Yes | 15 (18%) | 23 (21%) |
| No | 57 (66%) | 71 (66%) |
| Censored | 14 (16%) | 14 (13%) |

The data are numbers of patients, or percentages of patients. TM=tamoxifen

Based on the computation of the genomic risk according to the rules of the formulas for a good or poor prognosis hereinbefore described, the signature of genes of the invention is a good predictor of DM at 5 and at 10 years. To that end, the relative risk (RR) between the patients with a good prognosis profile and the patients with a poor prognosis profile was calculated by means of a Cox proportional hazards analysis (Table 9).

Table 9: Univariate and multivariate Cox proportional hazards analysis in Loi dataset

| | | Dataset Loi (86 tumors) 1,3 | | | | Dataset Loi (108 tumors) 2,3 | | |
|---|---|---|---|---|---|---|---|---|
| | | RR [4] | CI 95% [4] | P [4] | | RR [4] | CI 95% [4] | P [4] |
| DM at 5 years | Univariate analysis | 19.9 | 2.6 to 154.4 | 4. 0E-03 | | 4.2 | 1.2 to 14.6 | 2. 1E-02 |
| | Multivariate analysis | 14.9 | 1.8 to 123.7 | 1. 2E-02 | | 3.9 | 1.1 to 14.7 | 4. 2E-02 |
| DM at 10 years | Univariate analysis | 8.2 | 2.3 to 28.7 | 1. 0E-03 | | 4.7 | 1.6 to 13.5 | 4. 5E-03 |
| | Multivariate analysis | 7.3 | 1.9 to 28.5 | 4. 0E-03 | | 4.2 | 1.3 to 13 | 1. 3E-02 |

[1] Only tumors with diameter ≤ 5 cm, without adjuvant treatment, N-, ER+ (all grades) and ER- (grades 1 and 2)
[2] Only tumors with diameter ≤ 5 cm, with adjuvant treatment, N+
[3] Stratified by hospital
[4] RR= relative risk; CI= Confidence Interval; P= probability

According to Univariate analysis, the RR of developing DM between both patient groups is 19.9 at 5 years (confidence interval CI 95% 2.6-154.4, P=0.004) or 8.2 at 10 years (CI 95% 2.3-28.7, P=0.001). If the hazards analysis is multivariate, i.e., including the clinical data (age of the patient, tumor size, tumor grade, ER status) in the model, a RR of 14.9 at 5 years (CI 95% 1.8-123.7, P=0.012) and of 7.3 at 10 years (CI 95% 1.9-28.5, P=0,004) is obtained. It is important to point out that these RR are statistically significant in multivariate analysis, which demonstrates that the signature of the genes identified in Tables 1 and 2 is a genomic predictor independent of other clinical parameters (Table 9 and Table 10).

Table 10: Multivariate proportional hazards analysis, DM at 5 years in Loi dataset

| | N-, No Tamoxifen (n=86) | | | N+, Tamoxifen (n=108) | | |
|---|---|---|---|---|---|---|
| VARIABLE | RELATIVE RISK | CI* 95% | P VALUE | RELATIVE RISK | CI* 95% | P VALUE |
| Signature of poor prognosis (vs. good prognosis) | 14.9 | 1.8 to 123.7 | 0.012 | 3.9 | 1.1 to 14.6 | 0.042 |
| Age (<45, 45 to 65, >65) | 1.3 | 0.4 to 3.6 | 0.678 | 4.7 | 0.5 to 44.4 | 0.173 |
| Degree of tumor differentiation | 1.0 | 0.4 to 2.6 | 0.962 | 1.3 | 0.6 to 3.1 | 0.499 |
| Tumor size (in cm) | 2.0 | 1.0 to 4.0 | 0.063 | 1.0 | 0.5 to 2.0 | 0.918 |
| ER status | 0.9 | 0.3 to 3.0 | 0.881 | 1.2 | 0.7 to 1.9 | 0.548 |

* CI denotes confidence interval

The probability of survival was also calculated in both patient groups (Table 11). Thus, the good prognosis group has a probability of survival at 5 years of 96.10 (± 2.2), and 92.3% (± 4.3) at 10 years. The poor prognosis group has a probability of 70.1% (± 6.2) at 5 years and 49.2% (± 8.7) at 10 years. The differences of survival between both groups are considerable, being 26% at 5 years and 43% at 10 years.

Table 11: Probabilities of survival of prognosis subgroups in Loi dataset

| Dataset | Genomic group | Probability of DM at 5 years | Probability DM at 10 years |
|---|---|---|---|
| Dataset Loi (86 tumors)[1] | Good prognosis<br>Poor prognosis | 96.1 ± 2.2<br>70.1 ± 6.2 | 92.3 ± 4.3<br>49.2 ± 8.7 |
| Dataset Loi (108 tumors)[2] | Good prognosis<br>Poor prognosis | 94.2 ± 2.8<br>76.3 ± 4 | 88.8 ± 5.3<br>58.2 ± 6.1 |

[1] Only tumors with diameter ≤ 5 cm, without adjuvant treatment, N-, ER+ (all grades) and ER- (grades 1 and 2)
[2] Only tumors diameter ≤ 5 cm, with adjuvant treatment, N+

3.2. The genetic signature of the invention is a genomic predictor of distant metastasis in breast cancer patients with lymph node metastasis and who received hormone therapy with tamoxifen.

Then it was checked whether the predictive signature of the invention was valid for patients who, at the time of extracting the tumor, had lymph node metastasis (N+), and received hormone therapy, with a tumor diameter of ≤ 5 cm, but independently of the age of the patient (108 samples, see Table 8 with clinical characteristics). Similarly to what has been described in section 3.1, the patients were divided into two risk groups: poor prognosis and good prognosis. Univariate and multivariate Cox analysis was performed to check the relative risks of developing DM at 5 or at 10 years between both groups (Tables 9 and 10). The results show that the RR is 4.2 at 5 years (CI 95% 1.2-14.6, P=0.021), or 4.7 at 10 years (CI 95% 1.6-13.5, P=0.004) in Univariate analysis. When clinical parameters were included in the Cox model (age of the patient, tumor size, tumor grade), the genomic predictor proved to be independent of these parameters, maintaining the RR between the two prognosis groups, being 3.9 at 5 years (CI 95% 1.1-14.7, P=0.042) and 4.2 at 10 years (CI 95% 1.3-13, P=0.013) (Tables 9 and 10).

The probability of survival was also calculated in both patient groups (Table 11). Thus, the good prognosis group has a probability of survival at 5 years of 94.2% (± 2.8) and 88.8% (± 5.3) at 10 years. The poor prognosis group has a probability of 76.3% (± 4) at 5 years and 58.2% (± 6.1) at 10 years. The differences of survival between both groups are the 17.9% at 5 years and 30.6% at 10 years, which are also significant.

Finally, the prediction capacity of the genomic signature in the patient subgroup within the Loi study who, in the absence of local metastasis at the time of the extraction of the tumor, were treated with hormone therapy, with tumor diameter ≤ 5 cm, independently of the age of the patient (89 samples, all ER+) was analyzed. The results showed that despite the fact that the two groups defined by the genomic risk had a RR in Univariate analysis of about 2.5 at 5 years or 1.9 at 10 years, the differences were not statistically significant (data not shown).

Overall, the results show that the genetic signature of the invention is a good predictor of the risk of DM at 5 and 10 years in tumors smaller than 5 cm, ER+ tumors, ER- tumors (grade 1 and 2), in N- patients without hormone treatment, and in N+ patients treated with tamoxifen. Furthermore, in these patients the predictor is independent of the age of the patient, ER status, tumor grade, and tumor size.

EXAMPLE 4

Comparison of the genetic signature of the invention with clinical predictors

By means of Kaplan-Meier curves, survivals free of DM of the good or poor prognosis patient groups (Figure 2, 86 N-patients and not treated with tamoxifen; Figure 3, 108 N+ patients and treated with tamoxifen) were compared according to the criteria based on the genetic signature of the invention (Figures 2A and 3A), or according to the consensual clinical prediction criteria of St. Gallen (Goldhirsch A, et al. J Clin Oncol 2001;19(18):3817-3827) (Figures 2B and 3B), or of the NIH (National Institutes of Health, USA) (Eifel P, et al. J Natl Cancer Inst 2001;93(13):979-989) (Figures 2D and 3D) (see Table 12).

Table 12: Clinical criteria for receiving adjuvant chemotherapy

| | | |
|---|---|---|
| St. Gallen | tumor ≥ 2 cm | |

(continued)

| | | |
|---|---|---|
| | ER-<br>Grade 2 or 3<br>patient < 35 years | any of these criteria |
| NIH | tumor > 1 cm | |

The criteria of St. Gallen and of the NIH classify the patients as high risk or low risk based on several histological and clinical characteristics. This comparison shows that the prognostic signature of the invention assigns more patients to the low risk (or good prognosis) group than traditional methods do (56%, compared with 21% according to criteria of St. Gallen and 13% according to criteria of the NIH for patients not treated with tamoxifen and N-; 32%, compared with 10% according to criteria of St. Gallen and 2% according to criteria of the NIH for patients treated with tamoxifen and N+). The poor prognosis patients according to the genomic signature tend to have a higher proportion of DM than the poor prognosis patients according to the clinical criteria of St Gallen and NIH. This result indicates that both groups of clinical criteria used today mistakenly classify a clinically significant number of patients in the poor prognosis group. Furthermore, the poor prognosis group defined according to criteria of St. Gallen includes many patients who had a genomic signature of good prognosis and a good result (Figure 2C and Figure 3C). Similar subgroups were identified within the high risk group identified according to criteria of the NIH (Figure 2E and Figure 3E).

Given that both the St. Gallen and the NIH subgroups include patients that are poorly classified within the poor prognosis group among the patients not treated with tamoxifen and N- (subgroup of 86 patients), there would be patients that would be over-treated in current clinical practice. In addition, since all the N+ patients received hormone therapy (subgroup of 108 patients), it is not possible to determine how necessary genomic prediction would be in the absence of hormone treatment. However, the results show that the genetic signature of the invention is a good predictor of the response to treatment with tamoxifen in patients with lymph node metastasis.

**Claims**

1. An *in vitro* method for determining the prognosis of a subject diagnosed with breast cancer or for selecting the treatment of a subject diagnosed with breast cancer which comprises determining the expression levels of the genes identified in Table 1 and in Table 2 in a tumor tissue sample from said subject, wherein an increase of the expression of the genes identified in Table 1 and a decrease of the expression of the genes identified in Table 2 with respect to a reference value is indicative of a worse prognosis or of said subject having to be treated with chemotherapy.

2. The method according to claim 1, wherein the quantification of the expression levels of the genes identified in Tables 1 and 2 comprises the quantification of the messenger RNA (mRNA) of said genes, or a fragment of said mRNA, the complementary DNA (cDNA) of said genes, or a fragment of said cDNA, or mixtures thereof.

3. The method according to any of claims 1 to 2, wherein the quantification of the expression levels of the genes identified in Tables 1 and 2 is performed by means of a quantitative multiplex polymerase chain reaction (PCR) or a DNA or RNA array.

4. The method according to any of claims 1 to 3, wherein the determination of the expression levels of the genes identified in Table 1 and Table 2 is performed by means of a DNA array comprising the probes identified in Tables 3 and 4.

5. The method according to any of claims 1 to 4, wherein the determination of said prognosis comprises a proportional hazards regression analysis of said prognosis depending on the expression levels of the genes identified in Table 1 and in Table 2.

6. The method according to claim 5, wherein said proportional hazards regression analysis is a Cox-type analysis.

7. The method according to claim 6, wherein distant metastasis is established in said Cox-type analysis as a prognostic variable.

8. The method according to claim 7, wherein said distant metastasis is distant metastasis at 5 or 10 years.

9. The method according to any of claims 6 to 8, wherein the determination of said prognosis is carried out by applying the following formula:

$$\sum_{i=1}^{40} s_i \cdot x_i + 39.2$$

wherein $x_i$ is the value of the expression level in log2 of each of said genes identified in Tables 1 and 2; and $s_i$ is the value of the Wald statistic of the Cox-type regression analysis of each of said genes identified in Tables 1 and 2 according to claims 6 to 8,
wherein if said value is greater than zero, then it is indicative of said patient presenting a worse prognosis or of said patient having to be treated with chemotherapy, and wherein if said value is less than zero, it is indicative of said patient presenting a good prognosis or of said patient not having to be treated with chemotherapy.

**10.** The method according to claim 1, wherein the quantification of the expression levels of the genes identified in Tables 1 and 2 comprises the quantification of the levels of protein encoded by said genes or of a variant thereof.

**11.** The method according to claim 10, wherein the quantification of the levels of protein is performed by means of Western blot, ELISA or a protein array.

**12.** A reagent capable of detecting the expression levels of the genes identified in Tables 1 and 2.

**13.** The reagent according to claim 12, wherein the reagent comprises

(i) a set of nucleic acids comprising the nucleotide sequences of the probes identified in Tables 1 and 2 or the products of their transcription, or
(ii) a set of antibodies or a fragment thereof capable of detecting an antigen, consisting of each antibody or fragment being capable of binding specifically to one of the proteins encoded by the genes the nucleotide sequences of which hybridize with the probes identified in Tables 1 and 2.

**14.** The reagent according to claim 13, wherein the nucleic acids are DNA, cDNA or RNA probes and/or primers.

**15.** A kit comprising at least one reagent according to any of claims 12 to 14.

**16.** The kit according to claim 15, wherein said kit is a DNA or RNA array comprising a set of nucleic acids, wherein said set of nucleic acids comprises the nucleotide sequences of the probes of Tables 1 and 2, or a fragment thereof, or the products of their transcription.

**17.** The kit according to claim 16, further comprising a nucleic acid molecule of one or several constitutive expression genes.

**18.** The kit according to claim 17 comprising a set of antibodies, wherein said set of antibodies consists of antibodies or fragments thereof capable of binding specifically with the proteins encoded by the genes identified in Tables 1 and 2 or any variant of said proteins.

**19.** The kit according to claim 18, further comprising antibodies or fragments thereof capable of binding specifically with the proteins encoded by one or several constitutive expression genes.

**20.** Use of a kit according to any of claims 15 to 19 for the prognosis of patients diagnosed with breast cancer or for selecting the treatment of a subject diagnosed with breast cancer.

**21.** A method for selecting genetic markers for predicting the tendency to develop metastasis of a primary tumor comprising the following steps:

i) determining the genes the expression of which is altered with respect to a reference value in a tumor sample from a genetically modified non-human animal showing a tendency to develop tumors spontaneously;
ii) identifying the homologous genes in humans corresponding to the genes identified in step i); and
iii) selecting those genes identified in step ii) the expression of which in primary tumor samples from patients who develop metastasis from said primary tumor is altered with respect to the expression of said genes in primary tumors of patients who do not develop metastasis.

**22.** The method according to claim 21, wherein said non-human animal is an animal in which the gene expression of the Tp53 gene is inhibited.

**23.** The method according to claim 22, wherein said animal further presents inhibited gene expression of the pRb gene.

**24.** The method according to any of claims 21 to 23, wherein the sample obtained in step (i) is an epidermal carcinoma sample.

**25.** The method according to any of claims 21 to 24, wherein said step iii) is carried out by means of a proportional hazards regression analysis.

**26.** The method according to claim 25, wherein said regression analysis is a Cox-type analysis.

**27.** The method according to claim 26, wherein said Cox-type method establishes distant metastasis as a prognostic variable.

**28.** The method according to claim 27, wherein said distant metastasis is distant metastasis at 5 or 10 years.

**29.** The method according to any of claims 21 to 28, wherein said primary tumors analyzed in step iii) are breast cancer or glioblastoma tumors.

**30.** The method according to any of claims 21 to 29, wherein the determination of the expression of said genes according to step iii) comprises the quantification of the messenger RNA (mRNA) of said genes, or a fragment of said mRNA, the complementary DNA (cDNA), or a fragment of said cDNA, or mixtures thereof.

**31.** The method according to claim 30, wherein the quantification of the expression levels of the genes according to step iii) is performed by means of a quantitative multiplex polymerase chain reaction (PCR) or a DNA or RNA array.

**32.** The method according to any of claims 21 to 29, wherein the quantification of the expression levels of the genes according to step iii) comprises the quantification of the levels of protein encoded by said genes.

**33.** The method according to claim 32, wherein the quantification of the levels of protein is performed by means of Western blot, ELISA or a protein array.

**A)** Desmedt dataset. DM at 5 years (n=191)

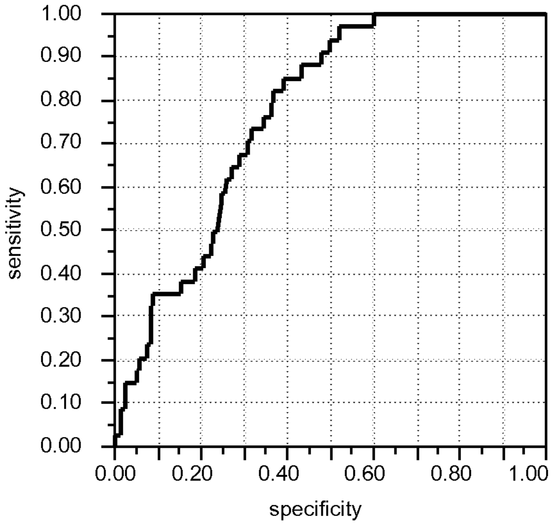


**B)** Desmedt dataset. DM at 10 years (n=191)

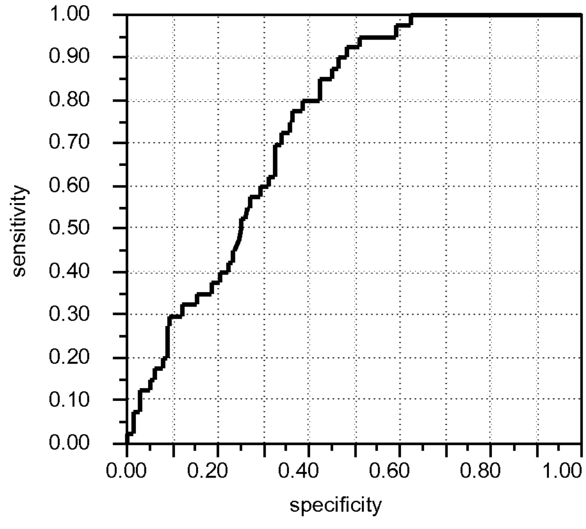

**C)** Desmedt dataset. DM at 5 years (n=142)

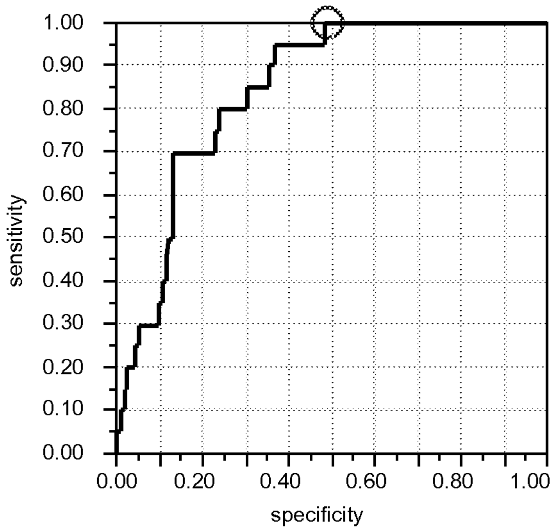


**D)** Desmedt dataset. DM at 10 years (n=142)

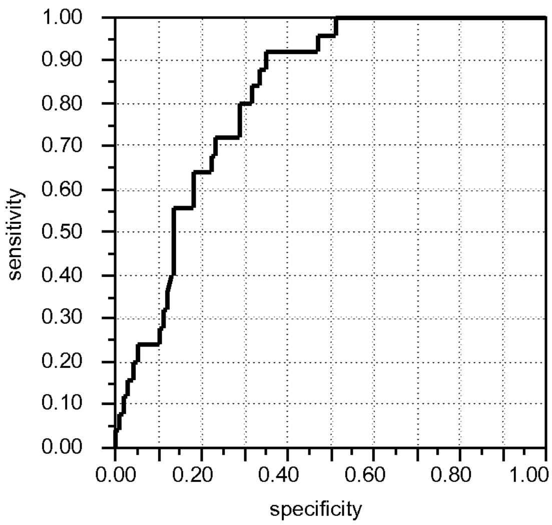

**E)** Desmedt dataset. Overall survival at 5 years (n=142)

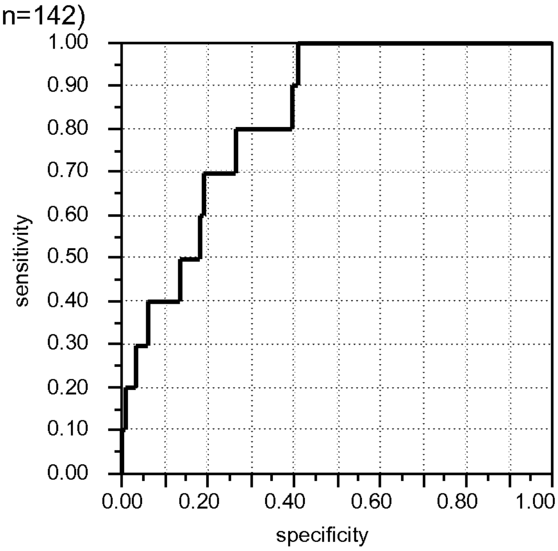


**F)** Desmedt dataset. Overall survival at 10 years (n=142)

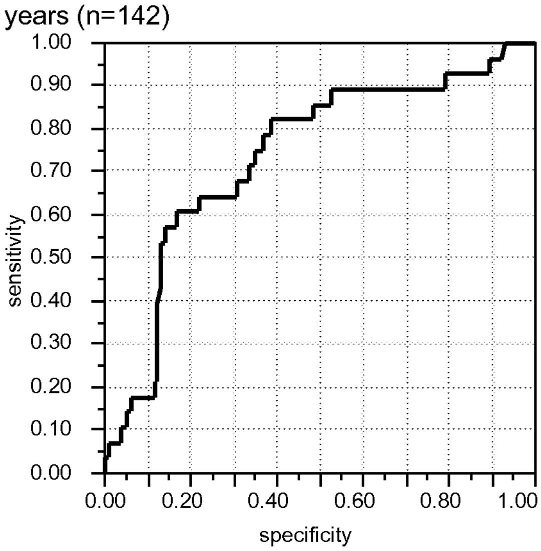


FIGURE 1

A) Genomic prediction criteria

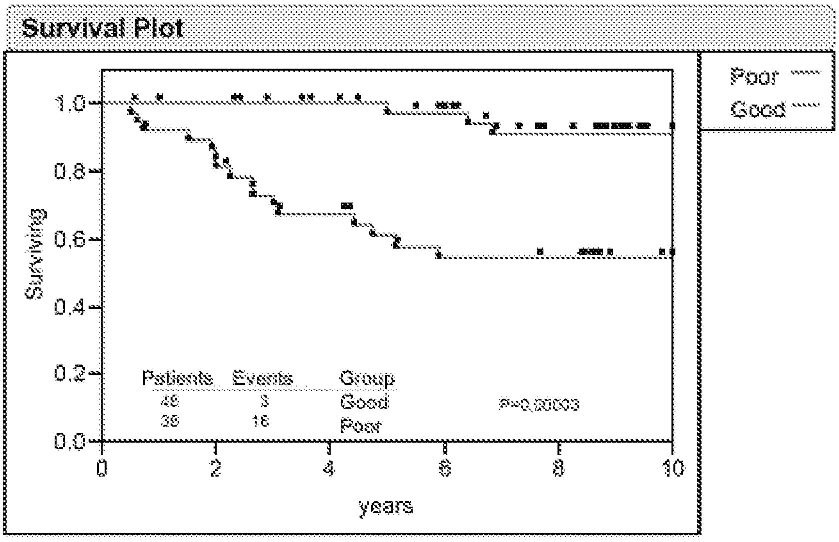


B) Clinical prediction criteria of St. Gallen

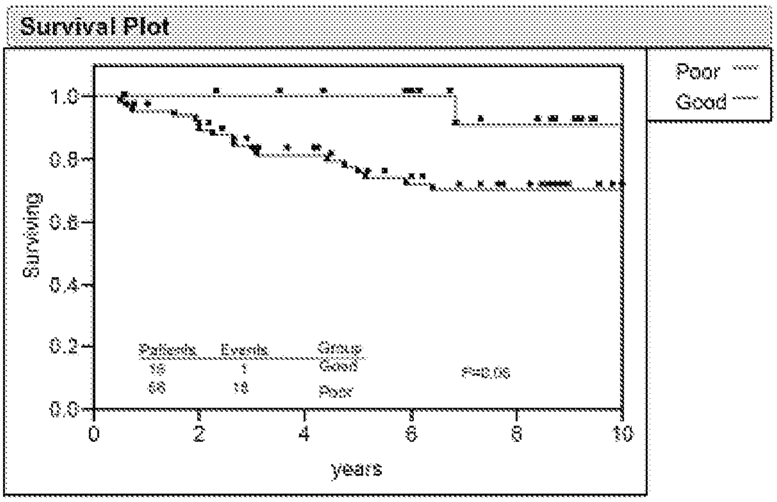

C) Genomic prediction of patients with a poor prognosis according to the clinical criteria of St. Gallen

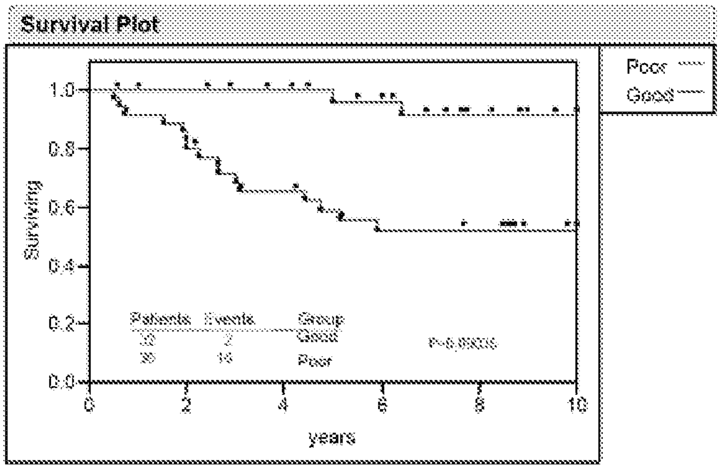


D) Clinical prediction criteria of NIH

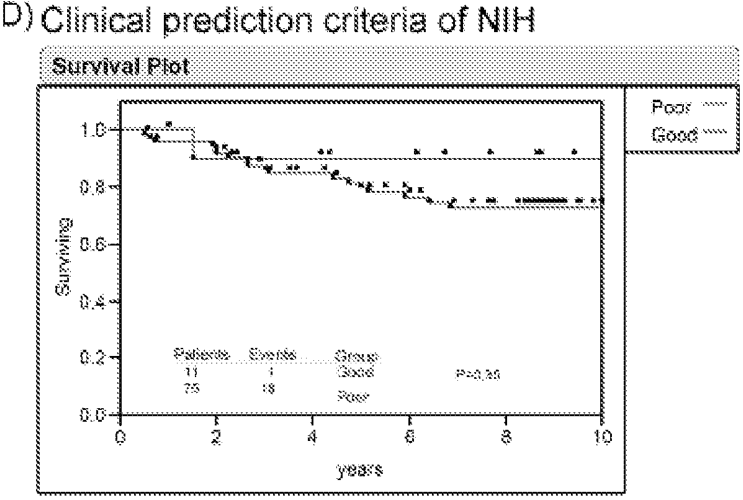

E) Genomic prediction of patients with poor prognosis according to clinical criteria of the NIH

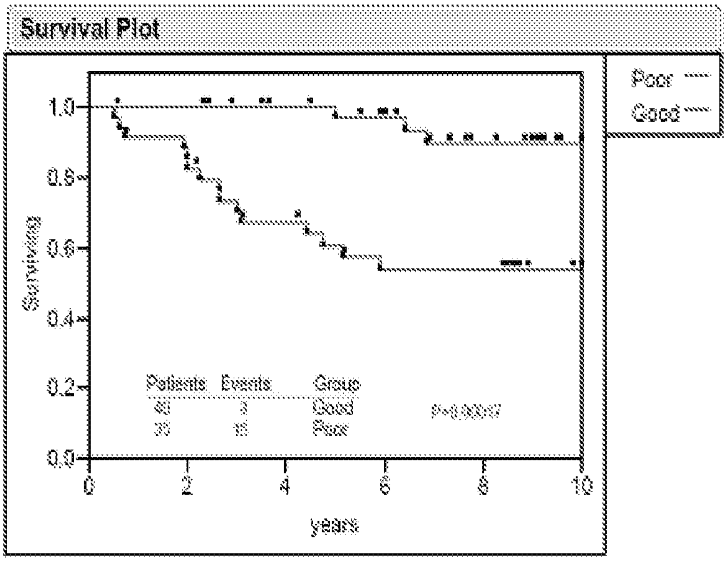


FIGURE 2

A) Genomic prediction criteria

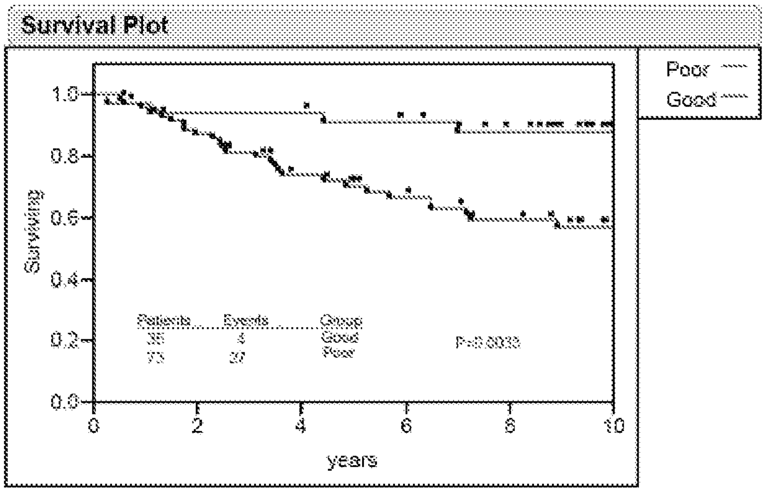


B) Clinical prediction criteria of St. Gallen

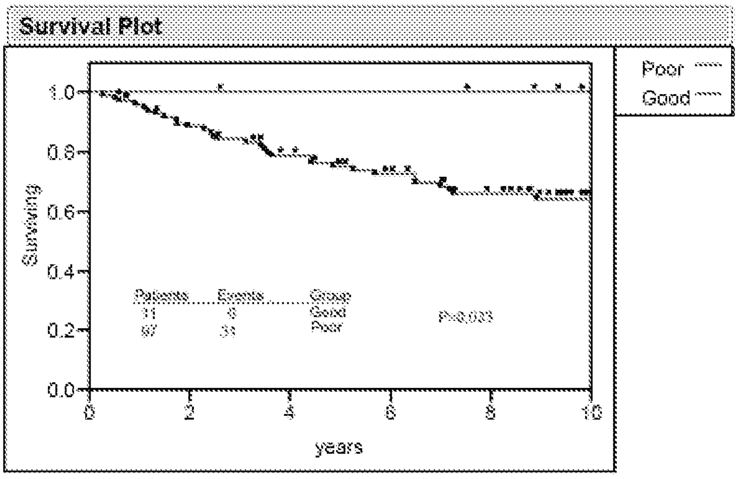

C) Genomic prediction of patients with a poor prognosis according to the clinical criteria of St. Gallen

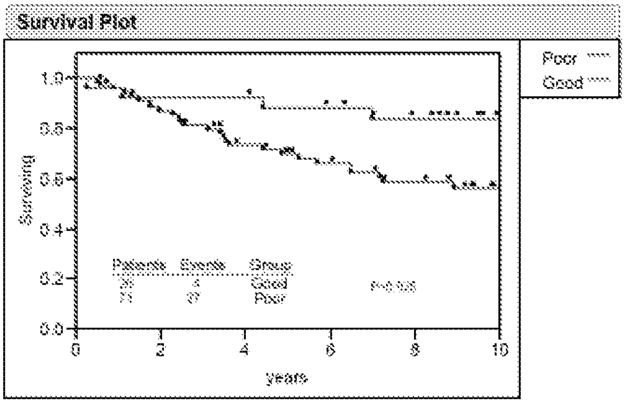


D) Clinical prediction criteria of the NIH

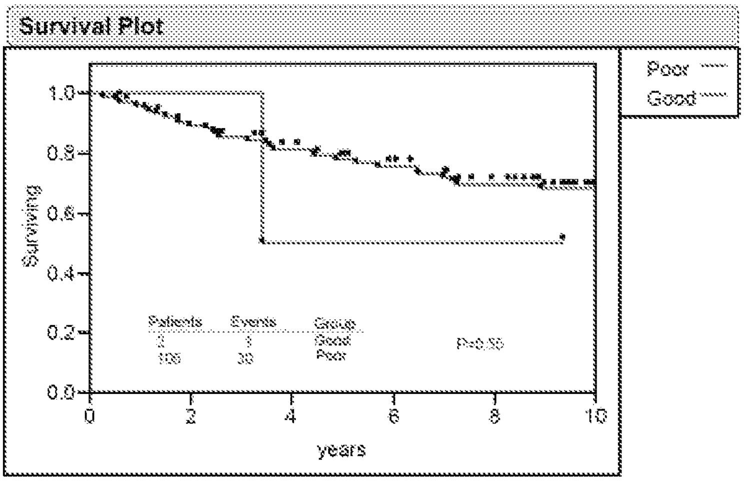

E) Genomic prediction of patients with a poor prognosis according to clinical criteria of the NIH

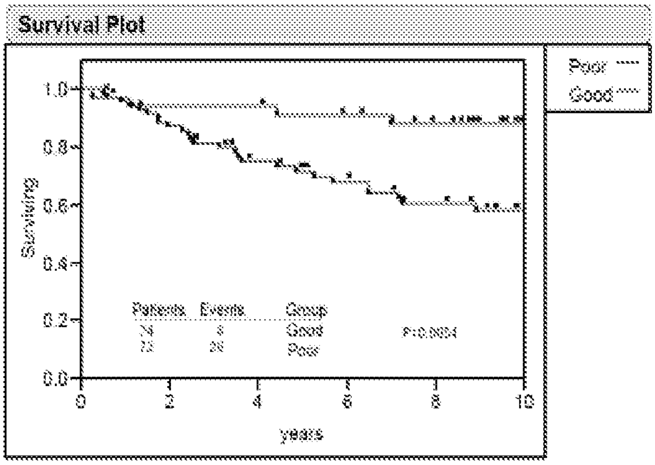


FIGURE 3

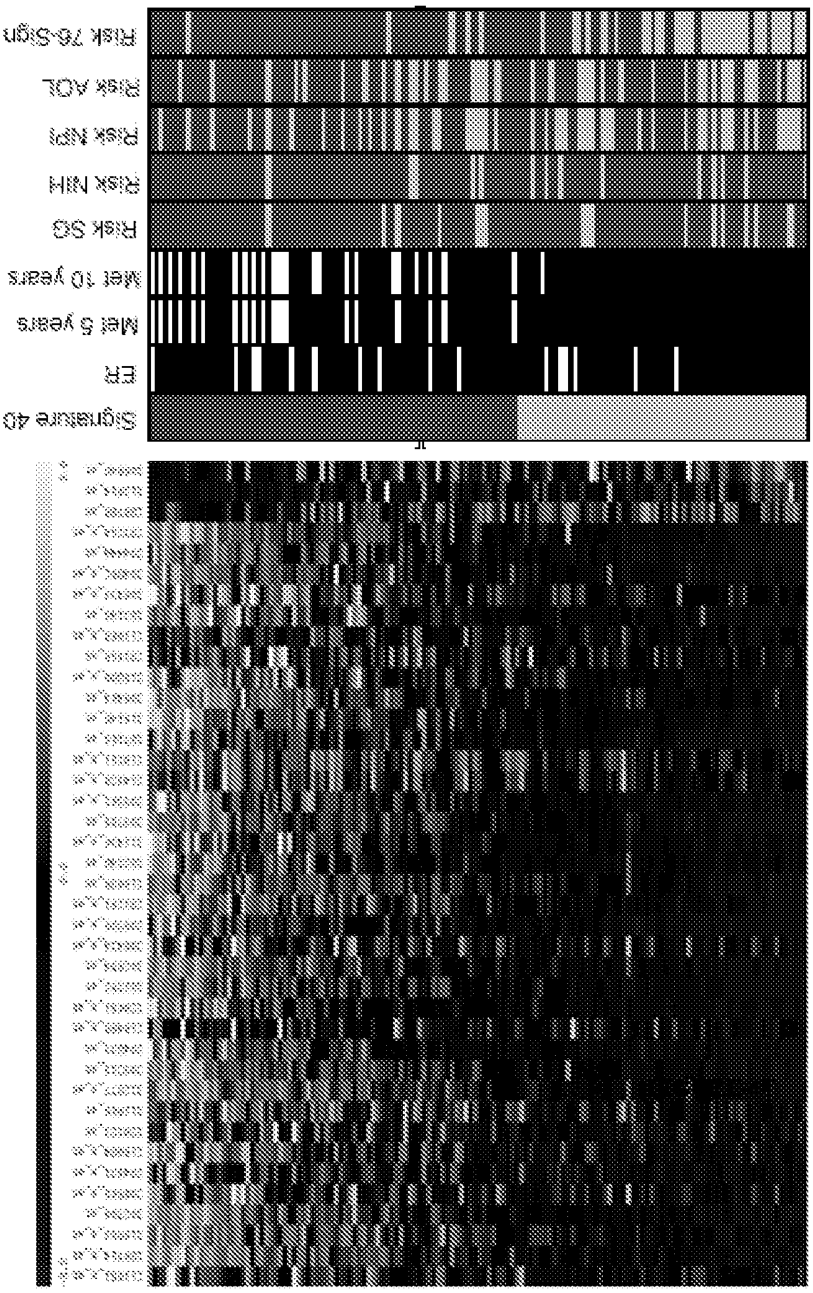
Risk 76-Sign
Risk AOL
Risk NPI
Risk NIH
Risk SG
Met 10 years
Met 5 years
ER
Signature 40

FIGURE 4

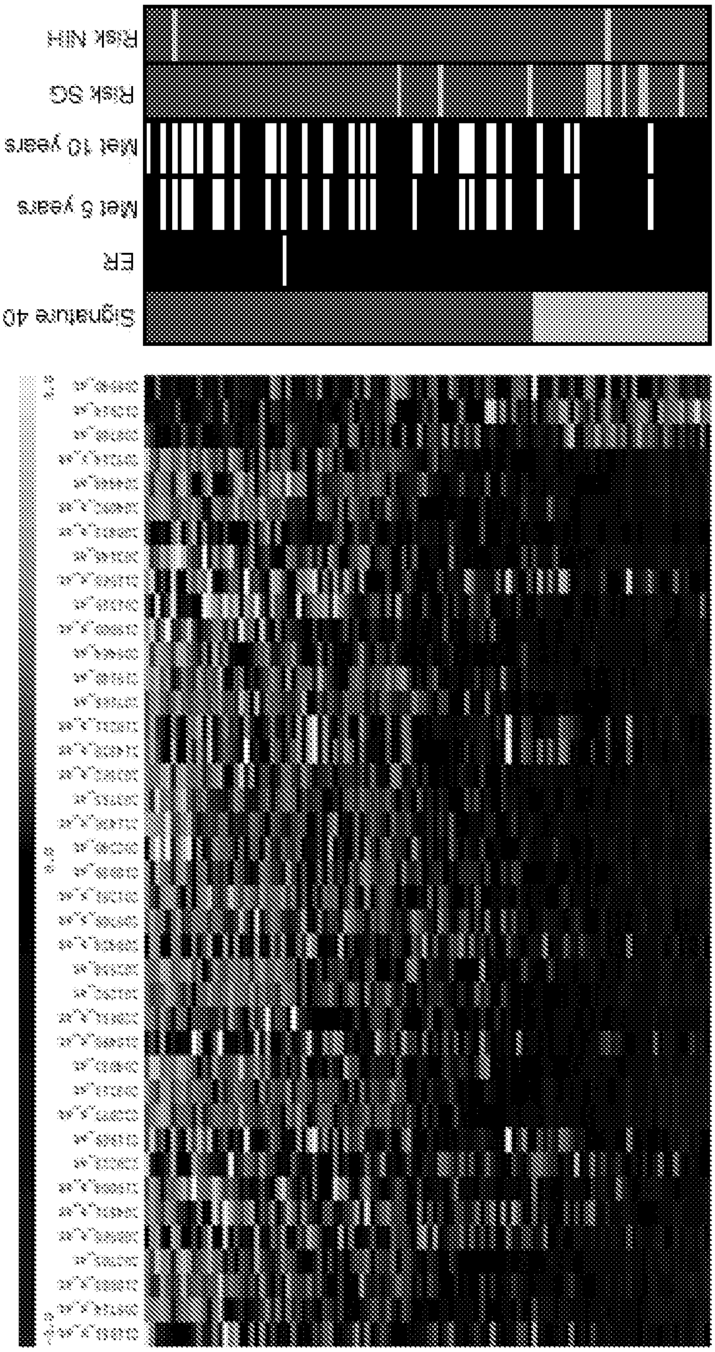


FIGURE 5

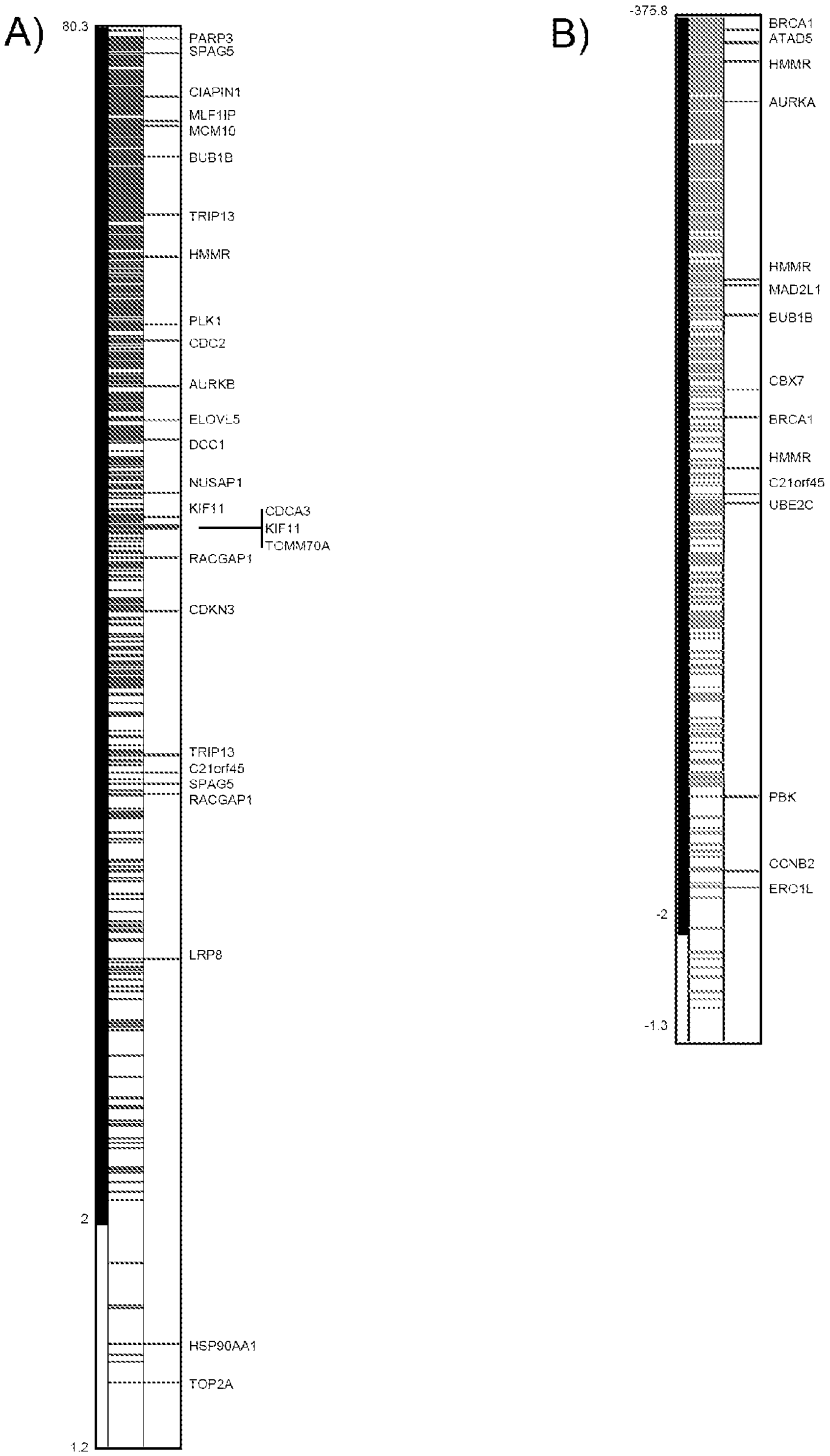
A)
80.3
PARP3
SPAG5
CIAPIN1
MLF1IP
MCM10
BUB1B
TRIP13
HMMR
PLK1
CDC2
AURKB
ELOVL5
DCC1
NUSAP1
KIF11
CDCA3
KIF11
TOMM70A
RACGAP1
CDKN3
TRIP13
C21orf45
SPAG5
RACGAP1
LRP8
2
HSP90AA1
TOP2A
1.2
B)
-375.8
BRCA1
ATAD5
HMMR
AURKA
HMMR
MAD2L1
BUB1B
CBX7
BRCA1
HMMR
C21orf45
UBE2C
PBK
CCNB2
ERO1L
-2
-1.3

C)

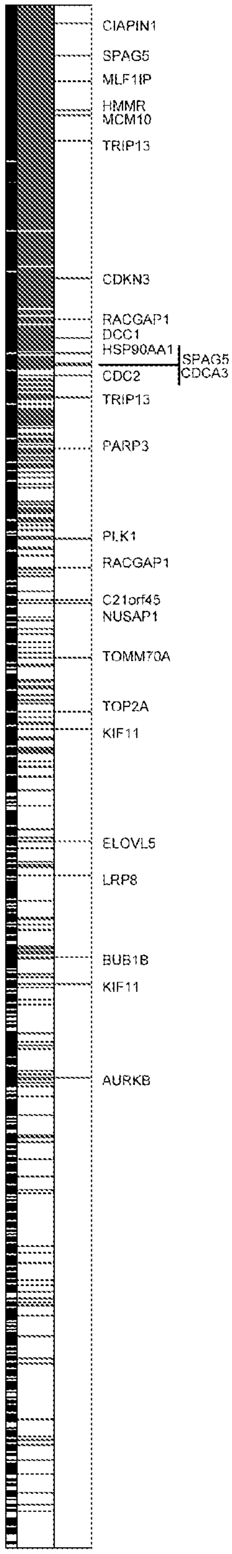
CIAPIN1
SPAG5
MLF1IP
HMMR
MCM10
TRIP13
CDKN3
RACGAP1
DCC1
HSP90AA1
SPAG5
CDCA3
CDC2
TRIP13
PARP3
PLK1
RACGAP1
C21orf45
NUSAP1
TOMM70A
TOP2A
KIF11
ELOVL5
LRP8
BUB1B
KIF11
AURKB

E)

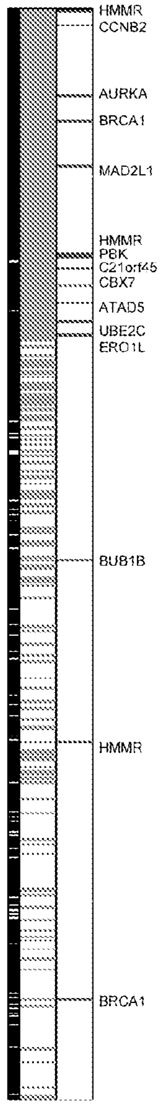
HMMR
CCNB2
AURKA
BRCA1
MAD2L1
HMMR
PBK
C21orf45
CBX7
ATAD5
UBE2C
ERO1L
BUB1B
HMMR
BRCA1

FIGURE 6

# INTERNATIONAL SEARCH REPORT

International application No.
PCT/ ES 2009/070268

A. CLASSIFICATION OF SUBJECT MATTER

see extra sheet

According to International Patent Classification (IPC) or to both national classification and IPC

B. FIELDS SEARCHED

Minimum documentation searched (classification system followed by classification symbols)
C12Q1/68, C12N15/12, G01N33/53

Documentation searched other than minimum documentation to the extent that such documents are included in the fields searched

Electronic data base consulted during the international search (name of data base and, where practicable, search terms used)

INVENES,EPODOC, WPI, TXTE, TXTF, MEDLINE, BIOSIS, NPL, XPESP, EMBASE, EMBL ALL.

C. DOCUMENTS CONSIDERED TO BE RELEVANT

| Category* | Citation of document, with indication, where appropriate, of the relevant passages | Relevant to claim No. |
|---|---|---|
| A | US2007099209. Paragraphs 17-23, 43 and 61. | 1-33 |
| A | MILLER W. R., LARIONOV A., RENSHAW L. , ANDERSON T. J., WHITE S., HAMPTON G., WALKER J. R., HO S., KRAUSE A., EVANS D. B., DIXON J. M. "Aromatase inhibitors - Gene discovery". Journal of Steroid Biochemistry & Molecular Biology 106 (2007) 130-142. Pages 131-132 and tables 2-4. | 1-33 |

☐ Further documents are listed in the continuation of Box C.    ☒ See patent family annex.

* Special categories of cited documents:

"A" document defining the general state of the art which is not considered to be of particular relevance.

"E" earlier document but published on or after the international filing date

"L" document which may throw doubts on priority claim(s) or which is cited to establish the publication date of another citation or other special reason (as specified)

"O" document referring to an oral disclosure use, exhibition, or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other documents , such combination being obvious to a person skilled in the art

"&" document member of the same patent family

| Date of the actual completion of the international search | Date of mailing of the international search report |
|---|---|
| 02 December 2009 (02.12.2009) | (07/12/2009) |
| Name and mailing address of the ISA/<br>O.E.P.M.<br>Paseo de la Castellana, 75 28071 Madrid, España.<br>Facsimile No. 34 91 3495304 | Authorized officer<br>M. Jesús García Bueno<br>Telephone No. +34 91 349 70 00 |

Form PCT/ISA/210 (second sheet) (July 2009)

## INTERNATIONAL SEARCH REPORT

Information on patent family members

International application No.

PCT/ ES 2009/070268

| Patent document cited in the search report | Publication date | Patent family member(s) | Publication date |
|---|---|---|---|
| US 2007099209 A | 03.05.2007 | WO 2006135886 A | 21.12.2006 |

Form PCT/ISA/210 (patent family annex) (July 2009)

**INTERNATIONAL SEARCH REPORT**

International application No.

PCT/ ES 2009/070268

CLASSIFICATION OF SUBJECT MATTER

***C12Q 1/68*** (2006.01)
***C12N 15/12*** (2006.01)
***G01N 33/53*** (2006.01)

Form PCT/ISA/210 (extra sheeet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02103320 A
- WO 2005083429 A
- WO 2005112973 A


### Non-patent literature cited in the description


- **Chomczynski et al.** *Anal. Biochem.,* 1987, vol. 162, 156
- **Chomczynski P.** *Biotechniques,* 1993, vol. 15, 532
- **Sambrook et al.** Molecular cloning: a Laboratory Manual. Cold Spring Harbor Laboratory Press, 2001, vol. 1-3
- **Wald A.** *Transactions of the American Mathematical Society,* 1943, vol. 54, 426-482
- **Silvey SD.** Annals of Mathematical Statistics. 1959, vol. 30, 389-407
- **Altschul S.F. et al.** Basic local alignment search tool. *J Mol Biol.,* 05 October 1990, vol. 215 (3), 403-10
- **Chen R et al.** *Nat Methods,* 2007, vol. 4 (11), 879
- **Martinez-Cruz AB et al.** *Cancer Res,* 2008, vol. 68 (3), 683-692
- **Bolstad BM et al.** *Bioinformatics,* 2003, vol. 19 (2), 185-193
- **Irizarry RA et al.** *Biostatistics,* 2003, vol. 4 (2), 249-264
- **Tusher VG et al.** *Proc Natl Acad Sci U S A,* 2001, vol. 98 (9), 5116-5121
- **Saeed AI et al.** *Biotechniques,* 2003, vol. 34 (2), 374-378
- **Benjamini Y ; Hochberg Y.** *Journal of the Royal Statistical Society B,* 1995, vol. 57, 289-300
- **Desmedt C. et al.** *Clin Cancer Res,* 2007, vol. 13 (11), 3207-3214
- **Loi S. et al.** *J Clin Oncol,* 2007, vol. 25 (10), 1239-1246
- **Vaquerizas JM. et al.** *Nucleic Acids Res,* 2005, 33
- **Wald A.** *Transactions of the American Mathematical Society,* 1943, vol. 54, 426-482
- **Goldhirsch A et al.** *J Clin Oncol,* 2001, vol. 19 (18), 3817-3827
- **Eifel P et al.** *J Natl Cancer Inst,* 2001, vol. 93 (13), 979-989

SEQUENCE LISTING

<110> CENTRO DE INVESTIGACIONES ENERGÉTICAS MEDIOAMBIENTALES Y TECNOLÓGICAS (CIEMAT)

<120> GENOMIC FINGERPRINT OF BREAST CANCER

<130> P3878EPPC

<140> EP 09772596.4
<141> 2009-07-01

<150> ES P200802024
<151> 2008-07-02

<160> 440

<170> PatentIn version 3.5

<210> 1
<211> 25
<212> DNA
<213> Artificial

<220>
<223> Probe 1 for determining the expression of the TOP2A gene

<400> 1
gagacttttt tgaactcaga cttaa 25

<210> 2
<211> 25
<212> DNA
<213> Artificial

<220>
<223> Probe 2 for determining the expression of the TOP2A gene

<400> 2
tggctcctag gaatgcttgg tgctg 25

<210> 3
<211> 25
<212> DNA
<213> Artificial

<220>
<223> Probe 3 for determining the expression of the TOP2A gene

<400> 3
cttggtgctg aatctgctaa actga 25

<210> 4
<211> 25
<212> DNA

<213>  Artificial

<220>
<223>  Probe 4 for determining the expression of the TOP2A gene

<400>  4
gaataatcag gctcgcttta tctta                                           25

<210>  5
<211>  25
<212>  DNA
<213>  Artificial

<220>
<223>  Probe 5 for determining the expression of the TOP2A gene

<400>  5
gatatgattc ggatcctgtg aaggc                                           25

<210>  6
<211>  25
<212>  DNA
<213>  Artificial

<220>
<223>  Probe 6 for determining the expression of the TOP2A gene

<400>  6
actccgtaac agattctgga ccaac                                           25

<210>  7
<211>  25
<212>  DNA
<213>  Artificial

<220>
<223>  Probe 7 for determining the expression of the TOP2A gene

<400>  7
gaccaacctt caactatctt cttga                                           25

<210>  8
<211>  25
<212>  DNA
<213>  Artificial

<220>
<223>  Probe 8 for determining the expression of the TOP2A gene

<400>  8
gaaagatgaa ctctgcaggc taaga                                           25

<210>  9

<211> 25
<212> DNA
<213> Artificial

<220>
<223> Probe 9 for determining the expression of the TOP2A gene

<400> 9
aagaacaaga gctggacaca ttaaa 25

<210> 10
<211> 25
<212> DNA
<213> Artificial

<220>
<223> Probe 10 for determining the expression of the TOP2A gene

<400> 10
aaagaaagag tccatcagat ttgtg 25

<210> 11
<211> 25
<212> DNA
<213> Artificial

<220>
<223> Probe 11 for determining the expression of the TOP2A gene

<400> 11
acaagatgaa caagtcggac ttcct 25

<210> 12
<211> 25
<212> DNA
<213> Artificial

<220>
<223> Probe 1 for determining the expression of the TOP2A-2 gene

<400> 12
tacagatact ctactacact cagcc 25

<210> 13
<211> 25
<212> DNA
<213> Artificial

<220>
<223> Probe 2 for determining the expression of the TOP2A-2 gene

<400> 13
ctactacact cagcctctta tgtgc 25

<210> 14
<211> 25
<212> DNA
<213> Artificial

<220>
<223> Probe 3 for determining the expression of the TOP2A-2 gene

<400> 14
gcctcttatg tgccaagttt ttctt 25

<210> 15
<211> 25
<212> DNA
<213> Artificial

<220>
<223> Probe 4 for determining the expression of the TOP2A-2 gene

<400> 15
tcatcttctc aaatcatcag aggcc 25

<210> 16
<211> 25
<212> DNA
<213> Artificial

<220>
<223> Probe 5 for determining the expression of the TOP2A-2 gene

<400> 16
actttggctg tgtctataac ttgac 25

<210> 17
<211> 25
<212> DNA
<213> Artificial

<220>
<223> Probe 6 for determining the expression of the TOP2A-2 gene

<400> 17
agtagttatg tgattatttc agctc 25

<210> 18
<211> 25
<212> DNA
<213> Artificial

<220>
<223> Probe 7 for determining the expression of the TOP2A-2 gene

<400> 18

actggattgc agaagactcg gggac 25

<210> 19
<211> 25
<212> DNA
<213> Artificial

<220>
<223> Probe 8 for determining the expression of the TOP2A-2 gene

<400> 19
gactcgggga caacatttga tccaa 25

<210> 20
<211> 25
<212> DNA
<213> Artificial

<220>
<223> Probe 9 for determining the expression of the TOP2A-2 gene

<400> 20
ttatattgat aaccatgctc agcaa 25

<210> 21
<211> 25
<212> DNA
<213> Artificial

<220>
<223> Probe 10 for determining the expression of the TOP2A-2 gene

<400> 21
attcattttg ggaaatctcc ataat 25

<210> 22
<211> 25
<212> DNA
<213> Artificial

<220>
<223> Probe 11 for determining the expression of the TOP2A-2 gene

<400> 22
taagacctgt ctacattgtt atatg 25

<210> 23
<211> 25
<212> DNA
<213> Artificial

<220>
<223> Probe 1 for determining the expression of the TOMM70A gene

<400> 23
taagggtgct taattctgtt gaaac 25

<210> 24
<211> 25
<212> DNA
<213> Artificial

<220>
<223> Probe 2 for determining the expression of the TOMM70A gene

<400> 24
ttgtctacaa atgctatctt tttta 25

<210> 25
<211> 25
<212> DNA
<213> Artificial

<220>
<223> Probe 3 for determining the expression of the TOMM70A gene

<400> 25
ggaagtttga gaccgctgca ttttg 25

<210> 26
<211> 25
<212> DNA
<213> Artificial

<220>
<223> Probe 4 for determining the expression of the TOMM70A gene

<400> 26
aaattatgca ccttctgata acccc 25

<210> 27
<211> 25
<212> DNA
<213> Artificial

<220>
<223> Probe 5 for determining the expression of the TOMM70A gene

<400> 27
aatgttctac atctctgaat gacct 25

<210> 28
<211> 25
<212> DNA
<213> Artificial

<220>
<223> Probe 6 for determining the expression of the TOMM70A gene

<400> 28
tctctgaatg acctctgact ttaaa 25

<210> 29
<211> 25
<212> DNA
<213> Artificial

<220>
<223> Probe 7 for determining the expression of the TOMM70A gene

<400> 29
tctcccttct ttcatcttgg ggttg 25

<210> 30
<211> 25
<212> DNA
<213> Artificial

<220>
<223> Probe 8 for determining the expression of the TOMM70A gene

<400> 30
gaagcatgtg ccattctata ctgtc 25

<210> 31
<211> 25
<212> DNA
<213> Artificial

<220>
<223> Probe 9 for determining the expression of the TOMM70A gene

<400> 31
gtgccattct atactgtcat tccaa 25

<210> 32
<211> 25
<212> DNA
<213> Artificial

<220>
<223> Probe 10 for determining the expression of the TOMM70A gene

<400> 32
aattctcatg gactattgcc tgttg 25

<210> 33
<211> 25
<212> DNA

<213>  Artificial

<220>
<223>  Probe 11 for determining the expression of the TOMM70A gene

<400>  33
ctgaaagctg catctgtctg tatct                                             25

<210>  34
<211>  25
<212>  DNA
<213>  Artificial

<220>
<223>  Probe 1 for determining the expression of the PLK1 gene

<400>  34
acgccgcgcg aaggtgatga gctcg                                             25

<210>  35
<211>  25
<212>  DNA
<213>  Artificial

<220>
<223>  Probe 2 for determining the expression of the PLK1 gene

<400>  35
acctcagcaa cggcagcgtg cagat                                             25

<210>  36
<211>  25
<212>  DNA
<213>  Artificial

<220>
<223>  Probe 3 for determining the expression of the PLK1 gene

<400>  36
atcaacttct tccaggatca cacca                                             25

<210>  37
<211>  25
<212>  DNA
<213>  Artificial

<220>
<223>  Probe 4 for determining the expression of the PLK1 gene

<400>  37
gatcacacca agctcatctt gtgcc                                             25

<210>  38

<211> 25
<212> DNA
<213> Artificial

<220>
<223> Probe 5 for determining the expression of the PLK1 gene

<400> 38
cactgatggc agccgtgacc tacat 25

<210> 39
<211> 25
<212> DNA
<213> Artificial

<220>
<223> Probe 6 for determining the expression of the PLK1 gene

<400> 39
gagaagcggg acttccgcac atacc 25

<210> 40
<211> 25
<212> DNA
<213> Artificial

<220>
<223> Probe 7 for determining the expression of the PLK1 gene

<400> 40
accgcctgag tctcctggag gagta 25

<210> 41
<211> 25
<212> DNA
<213> Artificial

<220>
<223> Probe 8 for determining the expression of the PLK1 gene

<400> 41
tacgcccgca ctatggtgga caagc 25

<210> 42
<211> 25
<212> DNA
<213> Artificial

<220>
<223> Probe 9 for determining the expression of the PLK1 gene

<400> 42
gtctcaaggc ctcctaatag ctgcc 25

<210> 43
<211> 25
<212> DNA
<213> Artificial

<220>
<223> Probe 10 for determining the expression of the PLK1 gene

<400> 43
gtggctgggc agagctgcat catcc 25

<210> 44
<211> 25
<212> DNA
<213> Artificial

<220>
<223> Probe 11 for determining the expression of the PLK1 gene

<400> 44
gtgtgggttc tacagacttg tcccc 25

<210> 45
<211> 25
<212> DNA
<213> Artificial

<220>
<223> Probe 1 for determining the expression of the CCNB2 gene

<400> 45
gccactacac ttcttaaggc gagca 25

<210> 46
<211> 25
<212> DNA
<213> Artificial

<220>
<223> Probe 2 for determining the expression of the CCNB2 gene

<400> 46
gcgagcatca aaagccgggg aggtt 25

<210> 47
<211> 25
<212> DNA
<213> Artificial

<220>
<223> Probe 3 for determining the expression of the CCNB2 gene

<400> 47

atggagctga ctctcatcga ctatg 25

<210> 48
<211> 25
<212> DNA
<213> Artificial

<220>
<223> Probe 4 for determining the expression of the CCNB2 gene

<400> 48
atatggtgca ttatcatcct tctaa 25

<210> 49
<211> 25
<212> DNA
<213> Artificial

<220>
<223> Probe 5 for determining the expression of the CCNB2 gene

<400> 49
atccttctaa ggtagcagca gctgc 25

<210> 50
<211> 25
<212> DNA
<213> Artificial

<220>
<223> Probe 6 for determining the expression of the CCNB2 gene

<400> 50
acttaactaa attcatcgcc atcaa 25

<210> 51
<211> 25
<212> DNA
<213> Artificial

<220>
<223> Probe 7 for determining the expression of the CCNB2 gene

<400> 51
caaaagccgt caaagacctt gcctc 25

<210> 52
<211> 25
<212> DNA
<213> Artificial

<220>
<223> Probe 8 for determining the expression of the CCNB2 gene

<400> 52
cttgcctccc cactgatagg aaggt 25

<210> 53
<211> 25
<212> DNA
<213> Artificial

<220>
<223> Probe 9 for determining the expression of the CCNB2 gene

<400> 53
gataggaagg tcctaggctg ccgtg 25

<210> 54
<211> 25
<212> DNA
<213> Artificial

<220>
<223> Probe 10 for determining the expression of the CCNB2 gene

<400> 54
gattttgtac atagtcctct ggtct 25

<210> 55
<211> 25
<212> DNA
<213> Artificial

<220>
<223> Probe 11 for determining the expression of the CCNB2 gene

<400> 55
agtcctctgg tctatctcat gaaac 25

<210> 56
<211> 25
<212> DNA
<213> Artificial

<220>
<223> Probe 1 for determining the expression of the UBEC2C gene

<400> 56
gccttccctg aatcagacaa ccttt 25

<210> 57
<211> 25
<212> DNA
<213> Artificial

<220>
<223> Probe 2 for determining the expression of the UBEC2C gene

<400> 57
gggtagggac catccatgga gcagc 25

<210> 58
<211> 25
<212> DNA
<213> Artificial

<220>
<223> Probe 3 for determining the expression of the UBEC2C gene

<400> 58
tataagctct cgctagagtt cccca 25

<210> 59
<211> 25
<212> DNA
<213> Artificial

<220>
<223> Probe 4 for determining the expression of the UBEC2C gene

<400> 59
caatgcgccc acagtgaagt tcctc 25

<210> 60
<211> 25
<212> DNA
<213> Artificial

<220>
<223> Probe 5 for determining the expression of the UBEC2C gene

<400> 60
gggtaacata tgcctggaca tcctg 25

<210> 61
<211> 25
<212> DNA
<213> Artificial

<220>
<223> Probe 6 for determining the expression of the UBEC2C gene

<400> 61
ggaaaagtgg tctgccctgt atgat 25

<210> 62
<211> 25
<212> DNA

<213>  Artificial

<220>
<223>  Probe 7 for determining the expression of the UBEC2C gene

<400>  62
atgatgtcag gaccattctg ctctc 25

<210>  63
<211>  25
<212>  DNA
<213>  Artificial

<220>
<223>  Probe 8 for determining the expression of the UBEC2C gene

<400>  63
tccatccaga gccttctagg agaac 25

<210>  64
<211>  25
<212>  DNA
<213>  Artificial

<220>
<223>  Probe 9 for determining the expression of the UBEC2C gene

<400>  64
tgatagtccc ttgaacacac atgct 25

<210>  65
<211>  25
<212>  DNA
<213>  Artificial

<220>
<223>  Probe 10 for determining the expression of the UBEC2C gene

<400>  65
gagctctgga aaaaccccac agctt 25

<210>  66
<211>  25
<212>  DNA
<213>  Artificial

<220>
<223>  Probe 11 for determining the expression of the UBEC2C gene

<400>  66
agatggtctg tcctttttgt gattt 25

<210>  67

```
<211>  25
<212>  DNA
<213>  Artificial

<220>
<223>  Probe 1 for determining the expression of the SPAG5 gene

<400>  67
gagtggcgag ctcataagcc ttaga                                          25


<210>  68
<211>  25
<212>  DNA
<213>  Artificial

<220>
<223>  Probe 2 for determining the expression of the SPAG5 gene

<400>  68
tagagaggag gtgacccacc ttacc                                          25


<210>  69
<211>  25
<212>  DNA
<213>  Artificial

<220>
<223>  Probe 3 for determining the expression of the SPAG5 gene

<400>  69
ctcacttcgg cgtgcggaga cagag                                          25


<210>  70
<211>  25
<212>  DNA
<213>  Artificial

<220>
<223>  Probe 4 for determining the expression of the SPAG5 gene

<400>  70
agagaccaaa gtgctccagg aggcc                                          25


<210>  71
<211>  25
<212>  DNA
<213>  Artificial

<220>
<223>  Probe 5 for determining the expression of the SPAG5 gene

<400>  71
gcctatggcc accaattgga tccag                                          25
```

<210> 72
<211> 25
<212> DNA
<213> Artificial

<220>
<223> Probe 6 for determining the expression of the SPAG5 gene

<400> 72
tcctagagga gaaccttcgg cgctc 25

<210> 73
<211> 25
<212> DNA
<213> Artificial

<220>
<223> Probe 7 for determining the expression of the SPAG5 gene

<400> 73
aaccttcggc gctctgacaa ggagt 25

<210> 74
<211> 25
<212> DNA
<213> Artificial

<220>
<223> Probe 8 for determining the expression of the SPAG5 gene

<400> 74
atttataaga ccctgctctc tattc 25

<210> 75
<211> 25
<212> DNA
<213> Artificial

<220>
<223> Probe 9 for determining the expression of the SPAG5 gene

<400> 75
ccctgctctc tattccagag gtggt 25

<210> 76
<211> 25
<212> DNA
<213> Artificial

<220>
<223> Probe 10 for determining the expression of the SPAG5 gene

<400> 76

gaaagccaga atttgtttca cctct 25

<210> 77
<211> 25
<212> DNA
<213> Artificial

<220>
<223> Probe 11 for determining the expression of the SPAG5 gene

<400> 77
taccccaata ccaagaccaa ctggc 25

<210> 78
<211> 25
<212> DNA
<213> Artificial

<220>
<223> Probe 1 for determining the expression of the CDC2 gene

<400> 78
tgctaagttc aagtttcgta atgct 25

<210> 79
<211> 25
<212> DNA
<213> Artificial

<220>
<223> Probe 2 for determining the expression of the CDC2 gene

<400> 79
tgaagtattt ttatgctctg aatgt 25

<210> 80
<211> 25
<212> DNA
<213> Artificial

<220>
<223> Probe 3 for determining the expression of the CDC2 gene

<400> 80
aaatgttctc atcagtttct tgcca 25

<210> 81
<211> 25
<212> DNA
<213> Artificial

<220>
<223> Probe 4 for determining the expression of the CDC2 gene

<400> 81
tgttaactat acaacctggc taaag 25

<210> 82
<211> 25
<212> DNA
<213> Artificial

<220>
<223> Probe 5 for determining the expression of the CDC2 gene

<400> 82
gatgaatatt tttctactgg tattt 25

<210> 83
<211> 25
<212> DNA
<213> Artificial

<220>
<223> Probe 6 for determining the expression of the CDC2 gene

<400> 83
caaagatcaa gggctgtccg caaca 25

<210> 84
<211> 25
<212> DNA
<213> Artificial

<220>
<223> Probe 7 for determining the expression of the CDC2 gene

<400> 84
aagggctgtc cgcaacaggg aagaa 25

<210> 85
<211> 25
<212> DNA
<213> Artificial

<220>
<223> Probe 8 for determining the expression of the CDC2 gene

<400> 85
gaaagctttt tgtctaagtg aattc 25

<210> 86
<211> 25
<212> DNA
<213> Artificial

<220>
<223> Probe 9 for determining the expression of the CDC2 gene

<400> 86
gtgaattctt atgccttggt cagag 25

<210> 87
<211> 25
<212> DNA
<213> Artificial

<220>
<223> Probe 10 for determining the expression of the CDC2 gene

<400> 87
cttatcttgg ctttcgagtc tgagt 25

<210> 88
<211> 25
<212> DNA
<213> Artificial

<220>
<223> Probe 11 for determining the expression of the CDC2 gene

<400> 88
gacatagtgt ttattagcag ccatc 25

<210> 89
<211> 25
<212> DNA
<213> Artificial

<220>
<223> Probe 1 for determining the expression of the CDC2-2 gene

<400> 89
ggggattgtg ttttgtcact ctaga 25

<210> 90
<211> 25
<212> DNA
<213> Artificial

<220>
<223> Probe 2 for determining the expression of the CDC2-2 gene

<400> 90
taaactggct gattttggcc ttgcc 25

<210> 91
<211> 25
<212> DNA

<213>  Artificial

<220>
<223>  Probe 3 for determining the expression of the CDC2-2 gene

<400>  91
ttggccttgc cagagctttt ggaat                                                   25

<210>  92
<211>  25
<212>  DNA
<213>  Artificial

<220>
<223>  Probe 4 for determining the expression of the CDC2-2 gene

<400>  92
gtaacactct ggtacagatc tccag                                                   25

<210>  93
<211>  25
<212>  DNA
<213>  Artificial

<220>
<223>  Probe 5 for determining the expression of the CDC2-2 gene

<400>  93
gtattgctgg ggtcagctcg ttact                                                   25

<210>  94
<211>  25
<212>  DNA
<213>  Artificial

<220>
<223>  Probe 6 for determining the expression of the CDC2-2 gene

<400>  94
agctcgttac tcaactccag ttgac                                                   25

<210>  95
<211>  25
<212>  DNA
<213>  Artificial

<220>
<223>  Probe 7 for determining the expression of the CDC2-2 gene

<400>  95
taggcaccat atttgctgaa ctagc                                                   25

<210>  96

<211> 25
<212> DNA
<213> Artificial

<220>
<223> Probe 8 for determining the expression of the CDC2-2 gene

<400> 96
gaaaccactt ttccatgggg attca 25

<210> 97
<211> 25
<212> DNA
<213> Artificial

<220>
<223> Probe 9 for determining the expression of the CDC2-2 gene

<400> 97
gattttcaga gctttgggca ctccc 25

<210> 98
<211> 25
<212> DNA
<213> Artificial

<220>
<223> Probe 10 for determining the expression of the CDC2-2 gene

<400> 98
aaaccaggaa gcctagcatc ccatg 25

<210> 99
<211> 25
<212> DNA
<213> Artificial

<220>
<223> Probe 11 for determining the expression of the CDC2-2 gene

<400> 99
aatggcttgg atttgctctc gaaaa 25

<210> 100
<211> 25
<212> DNA
<213> Artificial

<220>
<223> Probe 1 for determining the expression of the MAD2L1 gene

<400> 100
aaatgatact tactgaactg tgtgt 25

<210> 101
<211> 25
<212> DNA
<213> Artificial

<220>
<223> Probe 2 for determining the expression of the MAD2L1 gene

<400> 101
gtacctattt gacttaccat ggagt 25

<210> 102
<211> 25
<212> DNA
<213> Artificial

<220>
<223> Probe 3 for determining the expression of the MAD2L1 gene

<400> 102
ggaggttttt ttgtcaacat tgtga 25

<210> 103
<211> 25
<212> DNA
<213> Artificial

<220>
<223> Probe 4 for determining the expression of the MAD2L1 gene

<400> 103
aagctagatg ctttcctaaa tcaga 25

<210> 104
<211> 25
<212> DNA
<213> Artificial

<220>
<223> Probe 5 for determining the expression of the MAD2L1 gene

<400> 104
cagaatcttt gttaaggtcc tgaaa 25

<210> 105
<211> 25
<212> DNA
<213> Artificial

<220>
<223> Probe 6 for determining the expression of the MAD2L1 gene

<400> 105

aggtcctgaa agtaactcat aatct 25

<210> 106
<211> 25
<212> DNA
<213> Artificial

<220>
<223> Probe 7 for determining the expression of the MAD2L1 gene

<400> 106
attgctgtat agctcctttt gacct 25

<210> 107
<211> 25
<212> DNA
<213> Artificial

<220>
<223> Probe 8 for determining the expression of the MAD2L1 gene

<400> 107
ctccttttga ccttcatttc atgta 25

<210> 108
<211> 25
<212> DNA
<213> Artificial

<220>
<223> Probe 9 for determining the expression of the MAD2L1 gene

<400> 108
atttcatgta tagttttccc tattg 25

<210> 109
<211> 25
<212> DNA
<213> Artificial

<220>
<223> Probe 10 for determining the expression of the MAD2L1 gene

<400> 109
gttttcccta ttgaatcagt ttcca 25

<210> 110
<211> 25
<212> DNA
<213> Artificial

<220>
<223> Probe 11 for determining the expression of the MAD2L1 gene

<400> 110
atttgtactg tttaatgttc tgtga 25

<210> 111
<211> 25
<212> DNA
<213> Artificial

<220>
<223> Probe 1 for determining the expression of the BUB1B gene

<400> 111
ttctttgtgc ggattctgaa tgcca 25

<210> 112
<211> 25
<212> DNA
<213> Artificial

<220>
<223> Probe 2 for determining the expression of the BUB1B gene

<400> 112
tggggttttt gacactacat tccaa 25

<210> 113
<211> 25
<212> DNA
<213> Artificial

<220>
<223> Probe 3 for determining the expression of the BUB1B gene

<400> 113
gttaactagt cctggggctt tgctc 25

<210> 114
<211> 25
<212> DNA
<213> Artificial

<220>
<223> Probe 4 for determining the expression of the BUB1B gene

<400> 114
ggggctttgc tctttcagtg agcta 25

<210> 115
<211> 25
<212> DNA
<213> Artificial

<220>
<223> Probe 5 for determining the expression of the BUB1B gene

<400> 115
gagctaggca atcaagtctc acaga 25

<210> 116
<211> 25
<212> DNA
<213> Artificial

<220>
<223> Probe 6 for determining the expression of the BUB1B gene

<400> 116
gtctcacaga ttgctgcctc agagc 25

<210> 117
<211> 25
<212> DNA
<213> Artificial

<220>
<223> Probe 7 for determining the expression of the BUB1B gene

<400> 117
ggacacattt agatgcacta ccatt 25

<210> 118
<211> 25
<212> DNA
<213> Artificial

<220>
<223> Probe 8 for determining the expression of the BUB1B gene

<400> 118
cactaccatt gctgttctac ttttt 25

<210> 119
<211> 25
<212> DNA
<213> Artificial

<220>
<223> Probe 9 for determining the expression of the BUB1B gene

<400> 119
ggtacaggta tattttgacg tcact 25

<210> 120
<211> 25
<212> DNA

<213>  Artificial

<220>
<223>  Probe 10 for determining the expression of the BUB1B gene

<400>  120
ggccttgtct aacttttgtg aagaa                                          25

<210>  121
<211>  25
<212>  DNA
<213>  Artificial

<220>
<223>  Probe 11 for determining the expression of the BUB1B gene

<400>  121
gttctcttat gatcaccatg tattt                                          25

<210>  122
<211>  25
<212>  DNA
<213>  Artificial

<220>
<223>  Probe 1 for determining the expression of the TRIP13 gene

<400>  122
gaagaaccat cgaaacctgt ttgtt                                          25

<210>  123
<211>  25
<212>  DNA
<213>  Artificial

<220>
<223>  Probe 2 for determining the expression of the TRIP13 gene

<400>  123
aaatgcacac attactccag gtgga                                          25

<210>  124
<211>  25
<212>  DNA
<213>  Artificial

<220>
<223>  Probe 3 for determining the expression of the TRIP13 gene

<400>  124
ggtggcaatt gctttctgat atcag                                          25

<210>  125

<211> 25
<212> DNA
<213> Artificial

<220>
<223> Probe 4 for determining the expression of the TRIP13 gene

<400> 125
atcaagacat ggtcccattt gcagg 25

<210> 126
<211> 25
<212> DNA
<213> Artificial

<220>
<223> Probe 5 for determining the expression of the TRIP13 gene

<400> 126
gtgcagactc tgagtgttcc aggga 25

<210> 127
<211> 25
<212> DNA
<213> Artificial

<220>
<223> Probe 6 for determining the expression of the TRIP13 gene

<400> 127
gaaacacatg ctggacatcc cttgt 25

<210> 128
<211> 25
<212> DNA
<213> Artificial

<220>
<223> Probe 7 for determining the expression of the TRIP13 gene

<400> 128
catcccttgt aacccggtat gggcg 25

<210> 129
<211> 25
<212> DNA
<213> Artificial

<220>
<223> Probe 8 for determining the expression of the TRIP13 gene

<400> 129
ctgcattgct gggatgtttc tgccc 25

<210> 130
<211> 25
<212> DNA
<213> Artificial

<220>
<223> Probe 9 for determining the expression of the TRIP13 gene

<400> 130
ctgcccacgg ttttgtttgt gcaat 25

<210> 131
<211> 25
<212> DNA
<213> Artificial

<220>
<223> Probe 10 for determining the expression of the TRIP13 gene

<400> 131
ataggtcagt tactggtctc tttct 25

<210> 132
<211> 25
<212> DNA
<213> Artificial

<220>
<223> Probe 11 for determining the expression of the TRIP13 gene

<400> 132
ggtctctttc tgccgaatgt tatgt 25

<210> 133
<211> 25
<212> DNA
<213> Artificial

<220>
<223> Probe 1 for determining the expression of the AURKA gene

<400> 133
tgccctgacc ccgatcagtt aagga 25

<210> 134
<211> 25
<212> DNA
<213> Artificial

<220>
<223> Probe 2 for determining the expression of the AURKA gene

<400> 134

gaccccgatc agttaaggag ctgtg 25

<210> 135
<211> 25
<212> DNA
<213> Artificial

<220>
<223> Probe 3 for determining the expression of the AURKA gene

<400> 135
gagctgtgca ataaccttcc tagta 25

<210> 136
<211> 25
<212> DNA
<213> Artificial

<220>
<223> Probe 4 for determining the expression of the AURKA gene

<400> 136
gctgtgcaat aaccttccta gtacc 25

<210> 137
<211> 25
<212> DNA
<213> Artificial

<220>
<223> Probe 5 for determining the expression of the AURKA gene

<400> 137
aaagctgttg gaatgagtat gtgat 25

<210> 138
<211> 25
<212> DNA
<213> Artificial

<220>
<223> Probe 6 for determining the expression of the AURKA gene

<400> 138
ttgtattttt tctctggtgg cattc 25

<210> 139
<211> 25
<212> DNA
<213> Artificial

<220>
<223> Probe 7 for determining the expression of the AURKA gene

```
<400>  139
tttttctct ggtggcattc cttta                                              25


<210>  140
<211>  25
<212>  DNA
<213>  Artificial

<220>
<223>  Probe 8 for determining the expression of the AURKA gene

<400>  140
ttctctggtg gcattccttt aggaa                                             25


<210>  141
<211>  25
<212>  DNA
<213>  Artificial

<220>
<223>  Probe 9 for determining the expression of the AURKA gene

<400>  141
attcctttag gaatgctgtg tgtct                                             25


<210>  142
<211>  25
<212>  DNA
<213>  Artificial

<220>
<223>  Probe 10 for determining the expression of the AURKA gene

<400>  142
ttaaccactt atctcccata tgaga                                             25


<210>  143
<211>  25
<212>  DNA
<213>  Artificial

<220>
<223>  Probe 11 for determining the expression of the AURKA gene

<400>  143
cacttatctc ccatatgaga gtgtg                                             25


<210>  144
<211>  25
<212>  DNA
<213>  Artificial
```

<220>
<223> Probe 1 for determining the expression of the KIF11 gene

<400> 144
aagcccactt tagagtatac attgc 25

<210> 145
<211> 25
<212> DNA
<213> Artificial

<220>
<223> Probe 2 for determining the expression of the KIF11 gene

<400> 145
acattgctat tatgggagac caccc 25

<210> 146
<211> 25
<212> DNA
<213> Artificial

<220>
<223> Probe 3 for determining the expression of the KIF11 gene

<400> 146
acccagacat ctgactaatg gctct 25

<210> 147
<211> 25
<212> DNA
<213> Artificial

<220>
<223> Probe 4 for determining the expression of the KIF11 gene

<400> 147
tgactaatgg ctctgtgcca cactc 25

<210> 148
<211> 25
<212> DNA
<213> Artificial

<220>
<223> Probe 5 for determining the expression of the KIF11 gene

<400> 148
actccaagac ctgtgccttt tagag 25

<210> 149
<211> 25
<212> DNA

<213> Artificial

<220>
<223> Probe 6 for determining the expression of the KIF11 gene

<400> 149
gtatcttttt ctcgattcaa atctt 25

<210> 150
<211> 25
<212> DNA
<213> Artificial

<220>
<223> Probe 7 for determining the expression of the KIF11 gene

<400> 150
ttcaaatctt aacccttagg actct 25

<210> 151
<211> 25
<212> DNA
<213> Artificial

<220>
<223> Probe 8 for determining the expression of the KIF11 gene

<400> 151
aggactctgg tatttttgat ctggc 25

<210> 152
<211> 25
<212> DNA
<213> Artificial

<220>
<223> Probe 9 for determining the expression of the KIF11 gene

<400> 152
ttttgatctg gcaaccatat ttctg 25

<210> 153
<211> 25
<212> DNA
<213> Artificial

<220>
<223> Probe 10 for determining the expression of the KIF11 gene

<400> 153
gaataaattt tctgctcacg atgag 25

<210> 154

<211> 25
<212> DNA
<213> Artificial

<220>
<223> Probe 11 for determining the expression of the KIF11 gene

<400> 154
gagacatctg actttgatag ctaaa 25

<210> 155
<211> 25
<212> DNA
<213> Artificial

<220>
<223> Probe 1 for determining the expression of the BRCA1 gene

<400> 155
ttcaagaacc ggtttccaaa gacag 25

<210> 156
<211> 25
<212> DNA
<213> Artificial

<220>
<223> Probe 2 for determining the expression of the BRCA1 gene

<400> 156
atgtttattg ttgtagctct ggtat 25

<210> 157
<211> 25
<212> DNA
<213> Artificial

<220>
<223> Probe 3 for determining the expression of the BRCA1 gene

<400> 157
gctctggtat ataatccatt cctct 25

<210> 158
<211> 25
<212> DNA
<213> Artificial

<220>
<223> Probe 4 for determining the expression of the BRCA1 gene

<400> 158
taagacctct ggcatgaata tttca 25

<210> 159
<211> 25
<212> DNA
<213> Artificial

<220>
<223> Probe 5 for determining the expression of the BRCA1 gene

<400> 159
tgacagatcc caccaggaag gaagc 25

<210> 160
<211> 25
<212> DNA
<213> Artificial

<220>
<223> Probe 6 for determining the expression of the BRCA1 gene

<400> 160
tgctccctgt tgctgaaacc ataca 25

<210> 161
<211> 25
<212> DNA
<213> Artificial

<220>
<223> Probe 7 for determining the expression of the BRCA1 gene

<400> 161
aaaccataca gcttcataaa taatt 25

<210> 162
<211> 25
<212> DNA
<213> Artificial

<220>
<223> Probe 8 for determining the expression of the BRCA1 gene

<400> 162
cataaaccca ttatccagga ctgtt 25

<210> 163
<211> 25
<212> DNA
<213> Artificial

<220>
<223> Probe 9 for determining the expression of the BRCA1 gene

<400> 163

aggactgttt atagctgttg gaagg 25

<210> 164
<211> 25
<212> DNA
<213> Artificial

<220>
<223> Probe 10 for determining the expression of the BRCA1 gene

<400> 164
tggaaggact aggtcttccc tagcc 25

<210> 165
<211> 25
<212> DNA
<213> Artificial

<220>
<223> Probe 11 for determining the expression of the BRCA1 gene

<400> 165
agggcagtga agacttgatt gtaca 25

<210> 166
<211> 25
<212> DNA
<213> Artificial

<220>
<223> Probe 1 for determining the expression of the HMMR

<400> 166
atttacaggt tcttaggctc catcc 25

<210> 167
<211> 25
<212> DNA
<213> Artificial

<220>
<223> Probe 2 for determining the expression of the HMMR

<400> 167
aggctccatc ctgtttgtat gaaat 25

<210> 168
<211> 25
<212> DNA
<213> Artificial

<220>
<223> Probe 3 for determining the expression of the HMMR

<400> 168
taatctgtgg attggccttt aagcc 25

<210> 169
<211> 25
<212> DNA
<213> Artificial

<220>
<223> Probe 4 for determining the expression of the HMMR

<400> 169
gattggcctt taagcctgca ttctt 25

<210> 170
<211> 25
<212> DNA
<213> Artificial

<220>
<223> Probe 5 for determining the expression of the HMMR

<400> 170
gcctgcattc ttaacaaact cttca 25

<210> 171
<211> 25
<212> DNA
<213> Artificial

<220>
<223> Probe 6 for determining the expression of the HMMR

<400> 171
actctacatg taactatttc ttcag 25

<210> 172
<211> 25
<212> DNA
<213> Artificial

<220>
<223> Probe 7 for determining the expression of the HMMR

<400> 172
actatttctt cagagtttgt catat 25

<210> 173
<211> 25
<212> DNA
<213> Artificial

<220>
<223>  Probe 8 for determining the expression of the HMMR

<400>  173
gtttgtcata tactgcttgt catct                                                25

<210>  174
<211>  25
<212>  DNA
<213>  Artificial

<220>
<223>  Probe 9 for determining the expression of the HMMR

<400>  174
ctgcttgtca tctgcatgtc tactc                                                25

<210>  175
<211>  25
<212>  DNA
<213>  Artificial

<220>
<223>  Probe 10 for determining the expression of the HMMR

<400>  175
atctgcatgt ctactcagca tttga                                                25

<210>  176
<211>  25
<212>  DNA
<213>  Artificial

<220>
<223>  Probe 11 for determining the expression of the HMMR

<400>  176
tcagcatttg attaacattt gtgta                                                25

<210>  177
<211>  25
<212>  DNA
<213>  Artificial

<220>
<223>  Probe 1 for determining the expression of the CIAPIN1

<400>  177
gctgcatatc ttgacatatc ttgag                                                25

<210>  178
<211>  25
<212>  DNA

<213>  Artificial

<220>
<223>  Probe 2 for determining the expression of the CIAPIN1

<400>  178
atcttgagat tctgcatgtc ttgta                                          25

<210>  179
<211>  25
<212>  DNA
<213>  Artificial

<220>
<223>  Probe 3 for determining the expression of the CIAPIN1

<400>  179
gatgttggat agtcatccac gctca                                          25

<210>  180
<211>  25
<212>  DNA
<213>  Artificial

<220>
<223>  Probe 4 for determining the expression of the CIAPIN1

<400>  180
catccacgct cagtttggac cattg                                          25

<210>  181
<211>  25
<212>  DNA
<213>  Artificial

<220>
<223>  Probe 5 for determining the expression of the CIAPIN1

<400>  181
ggaggaactt agtgtcacgc acaaa                                          25

<210>  182
<211>  25
<212>  DNA
<213>  Artificial

<220>
<223>  Probe 6 for determining the expression of the CIAPIN1

<400>  182
caaatggggc tattcctacg cttag                                          25

<210>  183

<211> 25
<212> DNA
<213> Artificial

<220>
<223> Probe 7 for determining the expression of the CIAPIN1

<400> 183
tagaataggg cttgtctgcc cactt 25

<210> 184
<211> 25
<212> DNA
<213> Artificial

<220>
<223> Probe 8 for determining the expression of the CIAPIN1

<400> 184
gtctgcccac tttagaagag tccag 25

<210> 185
<211> 25
<212> DNA
<213> Artificial

<220>
<223> Probe 9 for determining the expression of the CIAPIN1

<400> 185
gaacgacaat acgtctctct gagca 25

<210> 186
<211> 25
<212> DNA
<213> Artificial

<220>
<223> Probe 10 for determining the expression of the CIAPIN1

<400> 186
gttcttgtta tccacccata tggac 25

<210> 187
<211> 25
<212> DNA
<213> Artificial

<220>
<223> Probe 11 for determining the expression of the CIAPIN1

<400> 187
tatggacttg gaatcaatct tgcca 25

<210> 188
<211> 25
<212> DNA
<213> Artificial

<220>
<223> Probe 1 for determining the expression of the LRP8

<400> 188
gaattttgac aacccagtct acagg 25

<210> 189
<211> 25
<212> DNA
<213> Artificial

<220>
<223> Probe 2 for determining the expression of the LRP8

<400> 189
tgctcagatt ggccatgtct atcct 25

<210> 190
<211> 25
<212> DNA
<213> Artificial

<220>
<223> Probe 3 for determining the expression of the LRP8

<400> 190
agatgatgga ctaccctgag gatgg 25

<210> 191
<211> 25
<212> DNA
<213> Artificial

<220>
<223> Probe 4 for determining the expression of the LRP8

<400> 191
ccttcgtgcc tcatggaatt cagtc 25

<210> 192
<211> 25
<212> DNA
<213> Artificial

<220>
<223> Probe 5 for determining the expression of the LRP8

<400> 192

tggaattcag tcccatgcac tacac 25

<210> 193
<211> 25
<212> DNA
<213> Artificial

<220>
<223> Probe 6 for determining the expression of the LRP8

<400> 193
gggtttctat atatgggtct gtgtg 25

<210> 194
<211> 25
<212> DNA
<213> Artificial

<220>
<223> Probe 7 for determining the expression of the LRP8

<400> 194
taactggttg cactacccat gagga 25

<210> 195
<211> 25
<212> DNA
<213> Artificial

<220>
<223> Probe 8 for determining the expression of the LRP8

<400> 195
ggaattcgtg gaatggctac tgctg 25

<210> 196
<211> 25
<212> DNA
<213> Artificial

<220>
<223> Probe 9 for determining the expression of the LRP8

<400> 196
gatgcacata accaaatggg ggcca 25

<210> 197
<211> 25
<212> DNA
<213> Artificial

<220>
<223> Probe 10 for determining the expression of the LRP8

<400> 197
atgggggcca atggcacagt acctt 25

<210> 198
<211> 25
<212> DNA
<213> Artificial

<220>
<223> Probe 11 for determining the expression of the LRP8

<400> 198
ggcacagtac cttactcatc attta 25

<210> 199
<211> 25
<212> DNA
<213> Artificial

<220>
<223> Probe 1 for determining the expression of the CIAPIN1-2

<400> 199
aatgggatgg gtttcttcac ctcat 25

<210> 200
<211> 25
<212> DNA
<213> Artificial

<220>
<223> Probe 2 for determining the expression of the CIAPIN1-2

<400> 200
aatgctgacc agaacgctct tgagc 25

<210> 201
<211> 25
<212> DNA
<213> Artificial

<220>
<223> Probe 3 for determining the expression of the CIAPIN1-2

<400> 201
tgagcccagg catcgttgag catta 25

<210> 202
<211> 25
<212> DNA
<213> Artificial

<220>
<223> Probe 4 for determining the expression of the CIAPIN1-2

<400> 202
gtctcatctc agcaatgctg ccacc 25

<210> 203
<211> 25
<212> DNA
<213> Artificial

<220>
<223> Probe 5 for determining the expression of the CIAPIN1-2

<400> 203
gtttactcca ttctttgtga cacga 25

<210> 204
<211> 25
<212> DNA
<213> Artificial

<220>
<223> Probe 6 for determining the expression of the CIAPIN1-2

<400> 204
cacgagtcaa gtggctcaca acctc 25

<210> 205
<211> 25
<212> DNA
<213> Artificial

<220>
<223> Probe 7 for determining the expression of the CIAPIN1-2

<400> 205
ggactcactc actggttgct gtgat 25

<210> 206
<211> 25
<212> DNA
<213> Artificial

<220>
<223> Probe 8 for determining the expression of the CIAPIN1-2

<400> 206
tgtgatgata tccagtgtcc ctctg 25

<210> 207
<211> 25
<212> DNA

<213>  Artificial

<220>
<223>  Probe 9 for determining the expression of the CIAPIN1-2

<400>  207
atccccaacc acatttgact gtagc                                          25

<210>  208
<211>  25
<212>  DNA
<213>  Artificial

<220>
<223>  Probe 10 for determining the expression of the CIAPIN1-2

<400>  208
gactgtagca ttgcatctgt gtcct                                          25

<210>  209
<211>  25
<212>  DNA
<213>  Artificial

<220>
<223>  Probe 11 for determining the expression of the CIAPIN1-2

<400>  209
atctgtgtcc tgttgtcatt tatgt                                          25

<210>  210
<211>  25
<212>  DNA
<213>  Artificial

<220>
<223>  Probe 1 for determining the expression of the AURKB

<400>  210
gaagagctgc acatttgacg agcag                                          25

<210>  211
<211>  25
<212>  DNA
<213>  Artificial

<220>
<223>  Probe 2 for determining the expression of the AURKB

<400>  211
tgacgagcag cgaacagcca cgatc                                          25

<210>  212

<211> 25
<212> DNA
<213> Artificial

<220>
<223> Probe 3 for determining the expression of the AURKB

<400> 212
gatgctctaa tgtactgcca tggga 25

<210> 213
<211> 25
<212> DNA
<213> Artificial

<220>
<223> Probe 4 for determining the expression of the AURKB

<400> 213
gccagaaaat ctgctcttag ggctc 25

<210> 214
<211> 25
<212> DNA
<213> Artificial

<220>
<223> Probe 5 for determining the expression of the AURKB

<400> 214
gaagacaatg tgtggcaccc tggac 25

<210> 215
<211> 25
<212> DNA
<213> Artificial

<220>
<223> Probe 6 for determining the expression of the AURKB

<400> 215
gaggggcgca tcgacaatga gaagg 25

<210> 216
<211> 25
<212> DNA
<213> Artificial

<220>
<223> Probe 7 for determining the expression of the AURKB

<400> 216
agctgctggt ggggaaccca tttga 25

<210> 217
<211> 25
<212> DNA
<213> Artificial

<220>
<223> Probe 8 for determining the expression of the AURKB

<400> 217
gaacccattt gagagtgcat cacac 25

<210> 218
<211> 25
<212> DNA
<213> Artificial

<220>
<223> Probe 9 for determining the expression of the AURKB

<400> 218
gcatcacaca acgagaccta tcgcc 25

<210> 219
<211> 25
<212> DNA
<213> Artificial

<220>
<223> Probe 10 for determining the expression of the AURKB

<400> 219
ctcatctcca aactgctcag gcata 25

<210> 220
<211> 25
<212> DNA
<213> Artificial

<220>
<223> Probe 11 for determining the expression of the AURKB

<400> 220
cattcactcg ggtgcgtgtg tttgt 25

<210> 221
<211> 25
<212> DNA
<213> Artificial

<220>
<223> Probe 1 for determining the expression of the HMMR-2

<400> 221

ttgccctgaa gaccccatta aaaga 25

<210> 222
<211> 25
<212> DNA
<213> Artificial

<220>
<223> Probe 2 for determining the expression of the HMMR-2

<400> 222
tacaaactgt taccgagctc ctatg 25

<210> 223
<211> 25
<212> DNA
<213> Artificial

<220>
<223> Probe 3 for determining the expression of the HMMR-2

<400> 223
gattatttca ttcgtcttgt tgtta 25

<210> 224
<211> 25
<212> DNA
<213> Artificial

<220>
<223> Probe 4 for determining the expression of the HMMR-2

<400> 224
cctttcgctg gctttccagc ttaga 25

<210> 225
<211> 25
<212> DNA
<213> Artificial

<220>
<223> Probe 5 for determining the expression of the HMMR-2

<400> 225
ccagcttaga atgcatctca tcaac 25

<210> 226
<211> 25
<212> DNA
<213> Artificial

<220>
<223> Probe 6 for determining the expression of the HMMR-2

<400> 226
catattatta tcctcctgtt ctgaa 25

<210> 227
<211> 25
<212> DNA
<213> Artificial

<220>
<223> Probe 7 for determining the expression of the HMMR-2

<400> 227
ccccagattc ttcagcttga tcctg 25

<210> 228
<211> 25
<212> DNA
<213> Artificial

<220>
<223> Probe 8 for determining the expression of the HMMR-2

<400> 228
cttttctagt ctgagcttct ttagc 25

<210> 229
<211> 25
<212> DNA
<213> Artificial

<220>
<223> Probe 9 for determining the expression of the HMMR-2

<400> 229
agctaggcta aaacaccttg gcttg 25

<210> 230
<211> 25
<212> DNA
<213> Artificial

<220>
<223> Probe 10 for determining the expression of the HMMR-2

<400> 230
accttggctt gttattgcct ctact 25

<210> 231
<211> 25
<212> DNA
<213> Artificial

<220>
<223>  Probe 11 for determining the expression of the HMMR-2

<400>  231
tcacttggtc ctacctatta tcctt    25

<210>  232
<211>  25
<212>  DNA
<213>  Artificial

<220>
<223>  Probe 1 for determining the expression of the CDKN3

<400>  232
tttctcggtt tatgtgctct tccag    25

<210>  233
<211>  25
<212>  DNA
<213>  Artificial

<220>
<223>  Probe 2 for determining the expression of the CDKN3

<400>  233
tagagtccca aaccttctgg atctc    25

<210>  234
<211>  25
<212>  DNA
<213>  Artificial

<220>
<223>  Probe 3 for determining the expression of the CDKN3

<400>  234
ggatctctac cagcaatgtg gaatt    25

<210>  235
<211>  25
<212>  DNA
<213>  Artificial

<220>
<223>  Probe 4 for determining the expression of the CDKN3

<400>  235
acccatcatc atccaatcgc agatg    25

<210>  236
<211>  25
<212>  DNA

<213>  Artificial

<220>
<223>  Probe 5 for determining the expression of the CDKN3

<400>  236
ctcctgacat agccagctgc tgtga 25

<210>  237
<211>  25
<212>  DNA
<213>  Artificial

<220>
<223>  Probe 6 for determining the expression of the CDKN3

<400>  237
tggaagagct tacaacctgc cttaa 25

<210>  238
<211>  25
<212>  DNA
<213>  Artificial

<220>
<223>  Probe 7 for determining the expression of the CDKN3

<400>  238
ggaggacttg ggagatcttg tcttg 25

<210>  239
<211>  25
<212>  DNA
<213>  Artificial

<220>
<223>  Probe 8 for determining the expression of the CDKN3

<400>  239
gacacaatat caccagagca agcca 25

<210>  240
<211>  25
<212>  DNA
<213>  Artificial

<220>
<223>  Probe 9 for determining the expression of the CDKN3

<400>  240
aagccataga cagcctgcga gacct 25

<210>  241

<211> 25
<212> DNA
<213> Artificial

<220>
<223> Probe 10 for determining the expression of the CDKN3

<400> 241
gaggatccgg ggcaatacag accat 25

<210> 242
<211> 25
<212> DNA
<213> Artificial

<220>
<223> Probe 11 for determining the expression of the CDKN3

<400> 242
attagctgca catctatcat caaga 25

<210> 243
<211> 25
<212> DNA
<213> Artificial

<220>
<223> Probe 1 for determining the expression of the HSP90AA1

<400> 243
tctgtatggc atgacaacta cttta 25

<210> 244
<211> 25
<212> DNA
<213> Artificial

<220>
<223> Probe 2 for determining the expression of the HSP90AA1

<400> 244
gatttctgtc tactaagtga tgctg 25

<210> 245
<211> 25
<212> DNA
<213> Artificial

<220>
<223> Probe 3 for determining the expression of the HSP90AA1

<400> 245
gtgatgctgt gataccttag gcact 25

<210> 246
<211> 25
<212> DNA
<213> Artificial

<220>
<223> Probe 4 for determining the expression of the HSP90AA1

<400> 246
gataccttag gcactaaagc agagc 25

<210> 247
<211> 25
<212> DNA
<213> Artificial

<220>
<223> Probe 5 for determining the expression of the HSP90AA1

<400> 247
aatgcttttt gagtttcatg ttggt 25

<210> 248
<211> 25
<212> DNA
<213> Artificial

<220>
<223> Probe 6 for determining the expression of the HSP90AA1

<400> 248
gattggggta acgtgcactg taaga 25

<210> 249
<211> 25
<212> DNA
<213> Artificial

<220>
<223> Probe 7 for determining the expression of the HSP90AA1

<400> 249
gtttagctgt caagccggat gccta 25

<210> 250
<211> 25
<212> DNA
<213> Artificial

<220>
<223> Probe 8 for determining the expression of the HSP90AA1

<400> 250

gccggatgcc taagtagacc aaatc 25

<210> 251
<211> 25
<212> DNA
<213> Artificial

<220>
<223> Probe 9 for determining the expression of the HSP90AA1

<400> 251
tgaagtgttc tgagctgtat cttga 25

<210> 252
<211> 25
<212> DNA
<213> Artificial

<220>
<223> Probe 10 for determining the expression of the HSP90AA1

<400> 252
gtattcgtta catcttgtag gatct 25

<210> 253
<211> 25
<212> DNA
<213> Artificial

<220>
<223> Probe 11 for determining the expression of the HSP90AA1

<400> 253
aggatctact ttttgaactt ttcat 25

<210> 254
<211> 25
<212> DNA
<213> Artificial

<220>
<223> Probe 1 for determining the expression of the BRCA1-2

<400> 254
aacaccacat cactttaact aatct 25

<210> 255
<211> 25
<212> DNA
<213> Artificial

<220>
<223> Probe 2 for determining the expression of the BRCA1-2 gene

<400> 255
gtagttagct atttctgggt gaccc 25

<210> 256
<211> 25
<212> DNA
<213> Artificial

<220>
<223> Probe 3 for determining the expression of the BRCA1-2 gene

<400> 256
accaacatgc ccacagatca actgg 25

<210> 257
<211> 25
<212> DNA
<213> Artificial

<220>
<223> Probe 4 for determining the expression of the BRCA1-2 gene

<400> 257
ggtacagctg tgtggtgctt ctgtg 25

<210> 258
<211> 25
<212> DNA
<213> Artificial

<220>
<223> Probe 5 for determining the expression of the BRCA1-2 gene

<400> 258
gaaggagctt tcatcattca ccctt 25

<210> 259
<211> 25
<212> DNA
<213> Artificial

<220>
<223> Probe 6 for determining the expression of the BRCA1-2 gene

<400> 259
attcaccctt ggcacaggtg tccac 25

<210> 260
<211> 25
<212> DNA
<213> Artificial

<220>
<223> Probe 7 for determining the expression of the BRCA1-2 gene

<400> 260
ggtgtccacc caattgtggt tgtgc 25

<210> 261
<211> 25
<212> DNA
<213> Artificial

<220>
<223> Probe 8 for determining the expression of the BRCA1-2 gene

<400> 261
ggttgtgcag ccagatgcct ggaca 25

<210> 262
<211> 25
<212> DNA
<213> Artificial

<220>
<223> Probe 9 for determining the expression of the BRCA1-2 gene

<400> 262
aggcacctgt ggtgacccga gagtg 25

<210> 263
<211> 25
<212> DNA
<213> Artificial

<220>
<223> Probe 10 for determining the expression of the BRCA1-2 gene

<400> 263
gtagcactct accagtgcca ggagc 25

<210> 264
<211> 25
<212> DNA
<213> Artificial

<220>
<223> Probe 11 for determining the expression of the BRCA1-2 gene

<400> 264
tgccaggagc tggacaccta cctga 25

<210> 265
<211> 25
<212> DNA

<213>  Artificial

<220>
<223>  Probe 1 for determining the expression of the HSP90AA1-2 gene

<400>  265
gtacgcagag ttttcatcat ggata    25

<210>  266
<211>  25
<212>  DNA
<213>  Artificial

<220>
<223>  Probe 2 for determining the expression of the HSP90AA1-2 gene

<400>  266
ggagctaatc cctgaatatc tgaac    25

<210>  267
<211>  25
<212>  DNA
<213>  Artificial

<220>
<223>  Probe 3 for determining the expression of the HSP90AA1-2 gene

<400>  267
ggtggtagac tcggaggatc tccct    25

<210>  268
<211>  25
<212>  DNA
<213>  Artificial

<220>
<223>  Probe 4 for determining the expression of the HSP90AA1-2 gene

<400>  268
tctccctcta aacatatccc gtgag    25

<210>  269
<211>  25
<212>  DNA
<213>  Artificial

<220>
<223>  Probe 5 for determining the expression of the HSP90AA1-2 gene

<400>  269
tgttaaggta ctacacatct gcctc    25

<210>  270

<211> 25
<212> DNA
<213> Artificial

<220>
<223> Probe 6 for determining the expression of the HSP90AA1-2 gene

<400> 270
gtttctctca aggactactg cacca 25

<210> 271
<211> 25
<212> DNA
<213> Artificial

<220>
<223> Probe 7 for determining the expression of the HSP90AA1-2 gene

<400> 271
ggaccaggta gctaactcag ccttt 25

<210> 272
<211> 25
<212> DNA
<213> Artificial

<220>
<223> Probe 8 for determining the expression of the HSP90AA1-2 gene

<400> 272
tcagcctttg tggaacgtct tcgga 25

<210> 273
<211> 25
<212> DNA
<213> Artificial

<220>
<223> Probe 9 for determining the expression of the HSP90AA1-2 gene

<400> 273
gaacgtcttc ggaaacatgg cttag 25

<210> 274
<211> 25
<212> DNA
<213> Artificial

<220>
<223> Probe 10 for determining the expression of the HSP90AA1-2 gene

<400> 274
gattgagccc attgatgagt actgt 25

<210> 275
<211> 25
<212> DNA
<213> Artificial

<220>
<223> Probe 11 for determining the expression of the HSP90AA1-2 gene

<400> 275
ggaagacttt agtgtcagtc accaa 25

<210> 276
<211> 25
<212> DNA
<213> Artificial

<220>
<223> Probe 1 for determining the expression of the HSP90AA1-3 gene

<400> 276
gagctgcata ttaaccttat accga 25

<210> 277
<211> 25
<212> DNA
<213> Artificial

<220>
<223> Probe 2 for determining the expression of the HSP90AA1-3 gene

<400> 277
aagatcgaac tctcactatt gtgga 25

<210> 278
<211> 25
<212> DNA
<213> Artificial

<220>
<223> Probe 3 for determining the expression of the HSP90AA1-3 gene

<400> 278
ggtactatcg ccaagtctgg gacca 25

<210> 279
<211> 25
<212> DNA
<213> Artificial

<220>
<223> Probe 4 for determining the expression of the HSP90AA1-3 gene

<400> 279

ggaagctttg caggctggtg cagat 25

<210> 280
<211> 25
<212> DNA
<213> Artificial

<220>
<223> Probe 5 for determining the expression of the HSP90AA1-3 gene

<400> 280
atctctatga ttggccagtt cggtg 25

<210> 281
<211> 25
<212> DNA
<213> Artificial

<220>
<223> Probe 6 for determining the expression of the HSP90AA1-3 gene

<400> 281
gttcggtgtt ggtttttatt ctgct 25

<210> 282
<211> 25
<212> DNA
<213> Artificial

<220>
<223> Probe 7 for determining the expression of the HSP90AA1-3 gene

<400> 282
atgagcagta cgcttgggag tcctc 25

<210> 283
<211> 25
<212> DNA
<213> Artificial

<220>
<223> Probe 8 for determining the expression of the HSP90AA1-3 gene

<400> 283
cctcagcagg gggatcattc acagt 25

<210> 284
<211> 25
<212> DNA
<213> Artificial

<220>
<223> Probe 9 for determining the expression of the HSP90AA1-3 gene

<400> 284
cacaggtgaa cctatgggtc gtgga 25

<210> 285
<211> 25
<212> DNA
<213> Artificial

<220>
<223> Probe 10 for determining the expression of the HSP90AA1-3 gene

<400> 285
gaacaaaagt tatcctacac ctgaa 25

<210> 286
<211> 25
<212> DNA
<213> Artificial

<220>
<223> Probe 11 for determining the expression of the HSP90AA1-3 gene

<400> 286
tggatatccc attactcttt ttgtg 25

<210> 287
<211> 25
<212> DNA
<213> Artificial

<220>
<223> Probe 1 for determining the expression of the HSP90AA1-4 gene

<400> 287
ccctgtagtt gacaattctg catgt 25

<210> 288
<211> 25
<212> DNA
<213> Artificial

<220>
<223> Probe 2 for determining the expression of the HSP90AA1-4 gene

<400> 288
aattctgcat gtactagtcc tctag 25

<210> 289
<211> 25
<212> DNA
<213> Artificial

<220>
<223> Probe 3 for determining the expression of the HSP90AA1-4 gene

<400> 289
gatggaagga tctctccaca gggct 25

<210> 290
<211> 25
<212> DNA
<213> Artificial

<220>
<223> Probe 4 for determining the expression of the HSP90AA1-4 gene

<400> 290
tctccacagg gcttgttttc caaag 25

<210> 291
<211> 25
<212> DNA
<213> Artificial

<220>
<223> Probe 5 for determining the expression of the HSP90AA1-4 gene

<400> 291
gagcaaagtt aaaagcctac ctaag 25

<210> 292
<211> 25
<212> DNA
<213> Artificial

<220>
<223> Probe 6 for determining the expression of the HSP90AA1-4 gene

<400> 292
aaagcctacc taagcatatc gtaaa 25

<210> 293
<211> 25
<212> DNA
<213> Artificial

<220>
<223> Probe 7 for determining the expression of the HSP90AA1-4 gene

<400> 293
gcatatcgta aagctgttca aaaat 25

<210> 294
<211> 25
<212> DNA

<213> Artificial

<220>
<223> Probe 8 for determining the expression of the HSP90AA1-4 gene

<400> 294
caaaaataac tcagacccag tcttg 25

<210> 295
<211> 25
<212> DNA
<213> Artificial

<220>
<223> Probe 9 for determining the expression of the HSP90AA1-4 gene

<400> 295
tcagacccag tcttgtggat ggaaa 25

<210> 296
<211> 25
<212> DNA
<213> Artificial

<220>
<223> Probe 10 for determining the expression of the HSP90AA1-4 gene

<400> 296
ggatggaaat gtagtgctcg agtca 25

<210> 297
<211> 25
<212> DNA
<213> Artificial

<220>
<223> Probe 11 for determining the expression of the HSP90AA1-4 gene

<400> 297
tgctcgagtc acattctgct taaag 25

<210> 298
<211> 25
<212> DNA
<213> Artificial

<220>
<223> Probe 1 for determining the expression of the NUSAP1 gene

<400> 298
aactgcagtc ttctgctagc caata 25

<210> 299

<211> 25
<212> DNA
<213> Artificial

<220>
<223> Probe 2 for determining the expression of the NUSAP1 gene

<400> 299
gggatagaaa ggccacctct tcact 25

<210> 300
<211> 25
<212> DNA
<213> Artificial

<220>
<223> Probe 3 for determining the expression of the NUSAP1 gene

<400> 300
cacctcttca ctctctatag aatat 25

<210> 301
<211> 25
<212> DNA
<213> Artificial

<220>
<223> Probe 4 for determining the expression of the NUSAP1 gene

<400> 301
tgtaccttcg ttcaaatatc ctcat 25

<210> 302
<211> 25
<212> DNA
<213> Artificial

<220>
<223> Probe 5 for determining the expression of the NUSAP1 gene

<400> 302
tcctcatgta attgccatct gtcac 25

<210> 303
<211> 25
<212> DNA
<213> Artificial

<220>
<223> Probe 6 for determining the expression of the NUSAP1 gene

<400> 303
catctgtcac tcactatatt cacaa 25

<210> 304
<211> 25
<212> DNA
<213> Artificial

<220>
<223> Probe 7 for determining the expression of the NUSAP1 gene

<400> 304
actcattcta acattgctta cttaa 25

<210> 305
<211> 25
<212> DNA
<213> Artificial

<220>
<223> Probe 8 for determining the expression of the NUSAP1 gene

<400> 305
gctacatagc cctatcgaaa tgcga 25

<210> 306
<211> 25
<212> DNA
<213> Artificial

<220>
<223> Probe 9 for determining the expression of the NUSAP1 gene

<400> 306
ggctttgctt agtatcatgt ccatg 25

<210> 307
<211> 25
<212> DNA
<213> Artificial

<220>
<223> Probe 10 for determining the expression of the NUSAP1 gene

<400> 307
ccttcacctc agtggagctt ctgag 25

<210> 308
<211> 25
<212> DNA
<213> Artificial

<220>
<223> Probe 11 for determining the expression of the NUSAP1 gene

<400> 308

gttttatact gctcaagatc gtcat 25

<210> 309
<211> 25
<212> DNA
<213> Artificial

<220>
<223> Probe 1 for determining the expression of the ERO1L gene

<400> 309
tcaagttcca atctaaagtt ctttt 25

<210> 310
<211> 25
<212> DNA
<213> Artificial

<220>
<223> Probe 2 for determining the expression of the ERO1L gene

<400> 310
gagttttgtt gcccgtttta tgctt 25

<210> 311
<211> 25
<212> DNA
<213> Artificial

<220>
<223> Probe 3 for determining the expression of the ERO1L gene

<400> 311
gttgcccgtt ttatgcttga tgtgt 25

<210> 312
<211> 25
<212> DNA
<213> Artificial

<220>
<223> Probe 4 for determining the expression of the ERO1L gene

<400> 312
ctggaacttg aacgactggg ctgaa 25

<210> 313
<211> 25
<212> DNA
<213> Artificial

<220>
<223> Probe 5 for determining the expression of the ERO1L gene

<400> 313
tacccgaagt tcatttcctt tgtct 25

<210> 314
<211> 25
<212> DNA
<213> Artificial

<220>
<223> Probe 6 for determining the expression of the ERO1L gene

<400> 314
tcctttgtct ccctaaaact gaact 25

<210> 315
<211> 25
<212> DNA
<213> Artificial

<220>
<223> Probe 7 for determining the expression of the ERO1L gene

<400> 315
ggttttcatt agtggaagct cttca 25

<210> 316
<211> 25
<212> DNA
<213> Artificial

<220>
<223> Probe 8 for determining the expression of the ERO1L gene

<400> 316
ggggtttagg aatttatatc acatg 25

<210> 317
<211> 25
<212> DNA
<213> Artificial

<220>
<223> Probe 9 for determining the expression of the ERO1L gene

<400> 317
ctatatacct caaaatcgtg ccctc 25

<210> 318
<211> 25
<212> DNA
<213> Artificial

<220>
<223> Probe 10 for determining the expression of the ERO1L gene

<400> 318
gtgccctctt tacatatgtc ttatc 25

<210> 319
<211> 25
<212> DNA
<213> Artificial

<220>
<223> Probe 11 for determining the expression of the ERO1L gene

<400> 319
atgtcagttt acactgctgt atact 25

<210> 320
<211> 25
<212> DNA
<213> Artificial

<220>
<223> Probe 1 for determining the expression of the MLF1IP gene

<400> 320
aaacgtatga ttcatccagc cttcc 25

<210> 321
<211> 25
<212> DNA
<213> Artificial

<220>
<223> Probe 2 for determining the expression of the MLF1IP gene

<400> 321
aagaacactt ctgggagccg aaagc 25

<210> 322
<211> 25
<212> DNA
<213> Artificial

<220>
<223> Probe 3 for determining the expression of the MLF1IP gene

<400> 322
ggagccgaaa gccatctgcg aaata 25

<210> 323
<211> 25
<212> DNA

<213>  Artificial

<220>
<223>  Probe 4 for determining the expression of the MLF1IP gene

<400>  323
gccatctgcg aaatatcaac catca                                    25

<210>  324
<211>  25
<212>  DNA
<213>  Artificial

<220>
<223>  Probe 5 for determining the expression of the MLF1IP gene

<400>  324
caaccatcag ttagagaagc tcctt                                    25

<210>  325
<211>  25
<212>  DNA
<213>  Artificial

<220>
<223>  Probe 6 for determining the expression of the MLF1IP gene

<400>  325
gaagctcctt gaccagggat gagaa                                    25

<210>  326
<211>  25
<212>  DNA
<213>  Artificial

<220>
<223>  Probe 7 for determining the expression of the MLF1IP gene

<400>  326
gtgcctatag gaagactagt ctcat                                    25

<210>  327
<211>  25
<212>  DNA
<213>  Artificial

<220>
<223>  Probe 8 for determining the expression of the MLF1IP gene

<400>  327
aaatagcatc agtttgtcca atagt                                    25

<210>  328

<211> 25
<212> DNA
<213> Artificial

<220>
<223> Probe 9 for determining the expression of the MLF1IP gene

<400> 328
aggccataat catcttttct ggtta 25

<210> 329
<211> 25
<212> DNA
<213> Artificial

<220>
<223> Probe 10 for determining the expression of the MLF1IP gene

<400> 329
atgttgacac cttaatcggt cccag 25

<210> 330
<211> 25
<212> DNA
<213> Artificial

<220>
<223> Probe 11 for determining the expression of the MLF1IP gene

<400> 330
atcggtccca ggtatgagct ataat 25

<210> 331
<211> 25
<212> DNA
<213> Artificial

<220>
<223> Probe 1 for determining the expression of the DCC1 gene

<400> 331
tcaagtgagt gagttcccct ctact 25

<210> 332
<211> 25
<212> DNA
<213> Artificial

<220>
<223> Probe 2 for determining the expression of the DCC1 gene

<400> 332
gccttccacc caaactggaa gcctc 25

<210> 333
<211> 25
<212> DNA
<213> Artificial

<220>
<223> Probe 3 for determining the expression of the DCC1 gene

<400> 333
ggaagcctct aggtgctatc aatta 25

<210> 334
<211> 25
<212> DNA
<213> Artificial

<220>
<223> Probe 4 for determining the expression of the DCC1 gene

<400> 334
attggctgaa taattactcc tctgc 25

<210> 335
<211> 25
<212> DNA
<213> Artificial

<220>
<223> Probe 5 for determining the expression of the DCC1 gene

<400> 335
gaatactggc acaggcaatg ctcac 25

<210> 336
<211> 25
<212> DNA
<213> Artificial

<220>
<223> Probe 6 for determining the expression of the DCC1 gene

<400> 336
tggcacaggc aatgctcact cgaaa 25

<210> 337
<211> 25
<212> DNA
<213> Artificial

<220>
<223> Probe 7 for determining the expression of the DCC1 gene

<400> 337

tctagttggt tttggaatgc ttgat 25

<210> 338
<211> 25
<212> DNA
<213> Artificial

<220>
<223> Probe 8 for determining the expression of the DCC1 gene

<400> 338
agctaatgaa ctcatcacca ggaca 25

<210> 339
<211> 25
<212> DNA
<213> Artificial

<220>
<223> Probe 9 for determining the expression of the DCC1 gene

<400> 339
ggacagttgg agggggtagg ccgag 25

<210> 340
<211> 25
<212> DNA
<213> Artificial

<220>
<223> Probe 10 for determining the expression of the DCC1 gene

<400> 340
gtaggccgag gttaaatggt ccacg 25

<210> 341
<211> 25
<212> DNA
<213> Artificial

<220>
<223> Probe 11 for determining the expression of the DCC1 gene

<400> 341
tggtccacgt ttcaaaaatg ttaat 25

<210> 342
<211> 25
<212> DNA
<213> Artificial

<220>
<223> Probe 1 for determining the expression of the C21orf45 gene

<400> 342
agccaggagg acaccaactg catcc 25

<210> 343
<211> 25
<212> DNA
<213> Artificial

<220>
<223> Probe 2 for determining the expression of the C21orf45 gene

<400> 343
gtgtttcctg taatgtttct gtgga 25

<210> 344
<211> 25
<212> DNA
<213> Artificial

<220>
<223> Probe 3 for determining the expression of the C21orf45 gene

<400> 344
gcgtccttga gactttgtgc tgcgc 25

<210> 345
<211> 25
<212> DNA
<213> Artificial

<220>
<223> Probe 4 for determining the expression of the C21orf45 gene

<400> 345
tgctcactca atcttggcta cgtgt 25

<210> 346
<211> 25
<212> DNA
<213> Artificial

<220>
<223> Probe 5 for determining the expression of the C21orf45 gene

<400> 346
ggctacgtgt acagatgcac gccca 25

<210> 347
<211> 25
<212> DNA
<213> Artificial

<220>
<223>  Probe 6 for determining the expression of the C21orf45 gene

<400>  347
cagatgcacg cccaagaatc ttgat                                             25

<210>  348
<211>  25
<212>  DNA
<213>  Artificial

<220>
<223>  Probe 7 for determining the expression of the C21orf45 gene

<400>  348
gagagacttg ttttgcctca gtgtt                                             25

<210>  349
<211>  25
<212>  DNA
<213>  Artificial

<220>
<223>  Probe 8 for determining the expression of the C21orf45 gene

<400>  349
gttttgcctc agtgttgaag ccatt                                             25

<210>  350
<211>  25
<212>  DNA
<213>  Artificial

<220>
<223>  Probe 9 for determining the expression of the C21orf45 gene

<400>  350
gaaagttatg ttttagggtc ctctg                                             25

<210>  351
<211>  25
<212>  DNA
<213>  Artificial

<220>
<223>  Probe 10 for determining the expression of the C21orf45 gene

<400>  351
gggaggccga atccaaattg tcctt                                             25

<210>  352
<211>  25
<212>  DNA

<213>  Artificial

<220>
<223>  Probe 11 for determining the expression of the C21orf45 gene

<400>  352
aagctgaact ctagtctgtg tcctc    25

<210>  353
<211>  25
<212>  DNA
<213>  Artificial

<220>
<223>  Probe 1 for determining the expression of the PBK gene

<400>  353
agcatactat gcagcgttgg gaact    25

<210>  354
<211>  25
<212>  DNA
<213>  Artificial

<220>
<223>  Probe 2 for determining the expression of the PBK gene

<400>  354
cagcgttggg aactaggcca cctat    25

<210>  355
<211>  25
<212>  DNA
<213>  Artificial

<220>
<223>  Probe 3 for determining the expression of the PBK gene

<400>  355
tgaactcttc tctgtatgca ctaat    25

<210>  356
<211>  25
<212>  DNA
<213>  Artificial

<220>
<223>  Probe 4 for determining the expression of the PBK gene

<400>  356
agaccctaaa gatcgtcctt ctgct    25

<210>  357

<211>  25
<212>  DNA
<213>  Artificial

<220>
<223>  Probe 5 for determining the expression of the PBK gene

<400>  357
atgtctagtg atcatctcag ctgaa                                                    25

<210>  358
<211>  25
<212>  DNA
<213>  Artificial

<220>
<223>  Probe 6 for determining the expression of the PBK gene

<400>  358
gtgtggcttg cgtaaataac tgttt                                                    25

<210>  359
<211>  25
<212>  DNA
<213>  Artificial

<220>
<223>  Probe 7 for determining the expression of the PBK gene

<400>  359
gaggaccata gtttcttgtt aacat                                                    25

<210>  360
<211>  25
<212>  DNA
<213>  Artificial

<220>
<223>  Probe 8 for determining the expression of the PBK gene

<400>  360
aagcacttgg aattgtactg ggttt                                                    25

<210>  361
<211>  25
<212>  DNA
<213>  Artificial

<220>
<223>  Probe 9 for determining the expression of the PBK gene

<400>  361
gtactttgat actgctcatg ctgac                                                    25

<210> 362
<211> 25
<212> DNA
<213> Artificial

<220>
<223> Probe 10 for determining the expression of the PBK gene

<400> 362
tgctcatgct gacttaaaac actag 25

<210> 363
<211> 25
<212> DNA
<213> Artificial

<220>
<223> Probe 11 for determining the expression of the PBK gene

<400> 363
ggatctactg acattagcac tttgt 25

<210> 364
<211> 25
<212> DNA
<213> Artificial

<220>
<223> Probe 1 for determining the expression of the ATAD5 gene

<400> 364
gggataaagc tagattcttc caaag 25

<210> 365
<211> 25
<212> DNA
<213> Artificial

<220>
<223> Probe 2 for determining the expression of the ATAD5 gene

<400> 365
caacctcaga ctgccagtga actta 25

<210> 366
<211> 25
<212> DNA
<213> Artificial

<220>
<223> Probe 3 for determining the expression of the ATAD5 gene

<400> 366

agatttctcg ggtggcatag acttt 25

<210> 367
<211> 25
<212> DNA
<213> Artificial

<220>
<223> Probe 4 for determining the expression of the ATAD5 gene

<400> 367
gagagtcgtc tttgcaatac tgtcc 25

<210> 368
<211> 25
<212> DNA
<213> Artificial

<220>
<223> Probe 5 for determining the expression of the ATAD5 gene

<400> 368
atactgtcct tataacaggg ccaac 25

<210> 369
<211> 25
<212> DNA
<213> Artificial

<220>
<223> Probe 6 for determining the expression of the ATAD5 gene

<400> 369
tgctgcagtg tatgcttgtg cccag 25

<210> 370
<211> 25
<212> DNA
<213> Artificial

<220>
<223> Probe 7 for determining the expression of the ATAD5 gene

<400> 370
tttgaagtga atgcctcttc ccagc 25

<210> 371
<211> 25
<212> DNA
<213> Artificial

<220>
<223> Probe 8 for determining the expression of the ATAD5 gene

<400> 371
ctcttcccag cgcagtggta gacaa 25

<210> 372
<211> 25
<212> DNA
<213> Artificial

<220>
<223> Probe 9 for determining the expression of the ATAD5 gene

<400> 372
gaagctactc agtcccatca agtag 25

<210> 373
<211> 25
<212> DNA
<213> Artificial

<220>
<223> Probe 10 for determining the expression of the ATAD5 gene

<400> 373
gctactacat aggcaagtca ccaag 25

<210> 374
<211> 25
<212> DNA
<213> Artificial

<220>
<223> Probe 11 for determining the expression of the ATAD5 gene

<400> 374
aactattttc cttaagccat aatgt 25

<210> 375
<211> 25
<212> DNA
<213> Artificial

<220>
<223> Probe 1 for determining the expression of the MCM10 gene

<400> 375
cagccttaaa taacccgaac ttcag 25

<210> 376
<211> 25
<212> DNA
<213> Artificial

<220>
<223> Probe 2 for determining the expression of the MCM10 gene

<400> 376
ggattggctg tgtattgtcc attga 25

<210> 377
<211> 25
<212> DNA
<213> Artificial

<220>
<223> Probe 3 for determining the expression of the MCM10 gene

<400> 377
tccattgatt cctgattgac gccgt 25

<210> 378
<211> 25
<212> DNA
<213> Artificial

<220>
<223> Probe 4 for determining the expression of the MCM10 gene

<400> 378
gttaagccca taagctttgc ctgct 25

<210> 379
<211> 25
<212> DNA
<213> Artificial

<220>
<223> Probe 5 for determining the expression of the MCM10 gene

<400> 379
taagctttgc ctgcttactt tctgc 25

<210> 380
<211> 25
<212> DNA
<213> Artificial

<220>
<223> Probe 6 for determining the expression of the MCM10 gene

<400> 380
gcttactttc tgccattggg ttggt 25

<210> 381
<211> 25
<212> DNA

<213> Artificial

<220>
<223> Probe 7 for determining the expression of the MCM10 gene

<400> 381
aaccaagtta tcattgtctt ttcta 25

<210> 382
<211> 25
<212> DNA
<213> Artificial

<220>
<223> Probe 8 for determining the expression of the MCM10 gene

<400> 382
gtcttttcta agctcagtgt ggatg 25

<210> 383
<211> 25
<212> DNA
<213> Artificial

<220>
<223> Probe 9 for determining the expression of the MCM10 gene

<400> 383
gtttatacga acacccagag gcaaa 25

<210> 384
<211> 25
<212> DNA
<213> Artificial

<220>
<223> Probe 10 for determining the expression of the MCM10 gene

<400> 384
atttggctta attctcactc caggt 25

<210> 385
<211> 25
<212> DNA
<213> Artificial

<220>
<223> Probe 11 for determining the expression of the MCM10 gene

<400> 385
agtagcttaa cttctgggct tcagt 25

<210> 386

<211> 25
<212> DNA
<213> Artificial

<220>
<223> Probe 1 for determining the expression of the CDCA3 gene

<400> 386
gcacggacac ctatgaagac cagca 25

<210> 387
<211> 25
<212> DNA
<213> Artificial

<220>
<223> Probe 2 for determining the expression of the CDCA3 gene

<400> 387
ccccaagccc actggtgaaa cagct 25

<210> 388
<211> 25
<212> DNA
<213> Artificial

<220>
<223> Probe 3 for determining the expression of the CDCA3 gene

<400> 388
ccagaggcac ctttatcttc tgaat 25

<210> 389
<211> 25
<212> DNA
<213> Artificial

<220>
<223> Probe 4 for determining the expression of the CDCA3 gene

<400> 389
ctgaattgga cttgcctctg ggtac 25

<210> 390
<211> 25
<212> DNA
<213> Artificial

<220>
<223> Probe 5 for determining the expression of the CDCA3 gene

<400> 390
ccagatcttc aggttctatg cgcaa 25

<210> 391
<211> 25
<212> DNA
<213> Artificial

<220>
<223> Probe 6 for determining the expression of the CDCA3 gene

<400> 391
gcaaggtact agggagatcc cccct 25

<210> 392
<211> 25
<212> DNA
<213> Artificial

<220>
<223> Probe 7 for determining the expression of the CDCA3 gene

<400> 392
tcctgcagga tgacaactcc cctgg 25

<210> 393
<211> 25
<212> DNA
<213> Artificial

<220>
<223> Probe 8 for determining the expression of the CDCA3 gene

<400> 393
tacgacaggg taagcggcct tcacc 25

<210> 394
<211> 25
<212> DNA
<213> Artificial

<220>
<223> Probe 9 for determining the expression of the CDCA3 gene

<400> 394
ggagccattc ttggaactgg acgac 25

<210> 395
<211> 25
<212> DNA
<213> Artificial

<220>
<223> Probe 10 for determining the expression of the CDCA3 gene

<400> 395

gagcaaggcc aggaccatga caagg 25

<210> 396
<211> 25
<212> DNA
<213> Artificial

<220>
<223> Probe 11 for determining the expression of the CDCA3 gene

<400> 396
aaaatcagca ctttcccttg gtgga 25

<210> 397
<211> 25
<212> DNA
<213> Artificial

<220>
<223> Probe 1 for determining the expression of the RACGAP1 gene

<400> 397
gtacaactcg tatttatctc tgatg 25

<210> 398
<211> 25
<212> DNA
<213> Artificial

<220>
<223> Probe 2 for determining the expression of the RACGAP1 gene

<400> 398
caatatatca tcctttggca tccca 25

<210> 399
<211> 25
<212> DNA
<213> Artificial

<220>
<223> Probe 3 for determining the expression of the RACGAP1 gene

<400> 399
agctacagtc attttttctt tgcac 25

<210> 400
<211> 25
<212> DNA
<213> Artificial

<220>
<223> Probe 4 for determining the expression of the RACGAP1 gene

<400>  400
ttttctttgc actttggatg ctgaa                                            25

<210>  401
<211>  25
<212>  DNA
<213>  Artificial

<220>
<223>  Probe 5 for determining the expression of the RACGAP1 gene

<400>  401
ggatgctgaa atttttccca tggaa                                            25

<210>  402
<211>  25
<212>  DNA
<213>  Artificial

<220>
<223>  Probe 6 for determining the expression of the RACGAP1 gene

<400>  402
ttcccatgga acatagccac atcta                                            25

<210>  403
<211>  25
<212>  DNA
<213>  Artificial

<220>
<223>  Probe 7 for determining the expression of the RACGAP1 gene

<400>  403
tctagataga tgtgagcttt ttctt                                            25

<210>  404
<211>  25
<212>  DNA
<213>  Artificial

<220>
<223>  Probe 8 for determining the expression of the RACGAP1 gene

<400>  404
aaacgatttt cttctgtaga atgtt                                            25

<210>  405
<211>  25
<212>  DNA
<213>  Artificial

<220>
<223> Probe 9 for determining the expression of the RACGAP1 gene

<400> 405
gaatgtttga cttcgtattg accct 25

<210> 406
<211> 25
<212> DNA
<213> Artificial

<220>
<223> Probe 10 for determining the expression of the RACGAP1 gene

<400> 406
acttcgtatt gacccttatc tgtaa 25

<210> 407
<211> 25
<212> DNA
<213> Artificial

<220>
<223> Probe 11 for determining the expression of the RACGAP1 gene

<400> 407
atctgtaaaa cacctatttg ggata 25

<210> 408
<211> 25
<212> DNA
<213> Artificial

<220>
<223> Probe 1 for determining the expression of the ELOVL5 gene

<400> 408
agatgtgttt agaacctctt gttta 25

<210> 409
<211> 25
<212> DNA
<213> Artificial

<220>
<223> Probe 2 for determining the expression of the ELOVL5 gene

<400> 409
tatcataaaa tcacatctca cacat 25

<210> 410
<211> 25
<212> DNA

<213>  Artificial

<220>
<223>  Probe 3 for determining the expression of the ELOVL5 gene

<400>  410
aatttagcct ctgaatacct tctcc    25

<210>  411
<211>  25
<212>  DNA
<213>  Artificial

<220>
<223>  Probe 4 for determining the expression of the ELOVL5 gene

<400>  411
ttcttggaac cactcatgac atatc    25

<210>  412
<211>  25
<212>  DNA
<213>  Artificial

<220>
<223>  Probe 5 for determining the expression of the ELOVL5 gene

<400>  412
gcacattcgt actataggga gccta    25

<210>  413
<211>  25
<212>  DNA
<213>  Artificial

<220>
<223>  Probe 6 for determining the expression of the ELOVL5 gene

<400>  413
gggagcctat tggttctcta ttagt    25

<210>  414
<211>  25
<212>  DNA
<213>  Artificial

<220>
<223>  Probe 7 for determining the expression of the ELOVL5 gene

<400>  414
ctctattagt cttgtgggtt ttctg    25

<210>  415

<211> 25
<212> DNA
<213> Artificial

<220>
<223> Probe 8 for determining the expression of the ELOVL5 gene

<400> 415
ggagtcatgg catctgttta cattt 25

<210> 416
<211> 25
<212> DNA
<213> Artificial

<220>
<223> Probe 9 for determining the expression of the ELOVL5 gene

<400> 416
gtatgtcttc attgctaggt actaa 25

<210> 417
<211> 25
<212> DNA
<213> Artificial

<220>
<223> Probe 10 for determining the expression of the ELOVL5 gene

<400> 417
ggtactaatt tgcagatgtc tttac 25

<210> 418
<211> 25
<212> DNA
<213> Artificial

<220>
<223> Probe 11 for determining the expression of the ELOVL5 gene

<400> 418
agctcacctg gatataccta cattg 25

<210> 419
<211> 25
<212> DNA
<213> Artificial

<220>
<223> Probe 1 for determining the expression of the PARP3 gene

<400> 419
tggcaccaac atggccgtgg tggcc 25

<210> 420
<211> 25
<212> DNA
<213> Artificial

<220>
<223> Probe 2 for determining the expression of the PARP3 gene

<400> 420
tctggtgggc gtgttggcaa gggca 25

<210> 421
<211> 25
<212> DNA
<213> Artificial

<220>
<223> Probe 3 for determining the expression of the PARP3 gene

<400> 421
gcaagggcat ctactttgcc tcaga 25

<210> 422
<211> 25
<212> DNA
<213> Artificial

<220>
<223> Probe 4 for determining the expression of the PARP3 gene

<400> 422
tcggctacat gttcctgggt gaggt 25

<210> 423
<211> 25
<212> DNA
<213> Artificial

<220>
<223> Probe 5 for determining the expression of the PARP3 gene

<400> 423
gggcagagag caccatatca acacg 25

<210> 424
<211> 25
<212> DNA
<213> Artificial

<220>
<223> Probe 6 for determining the expression of the PARP3 gene

<400> 424

tggcttcgac agtgtcattg cccga 25

<210> 425
<211> 25
<212> DNA
<213> Artificial

<220>
<223> Probe 7 for determining the expression of the PARP3 gene

<400> 425
atccgaccca ggacactgag ttgga 25

<210> 426
<211> 25
<212> DNA
<213> Artificial

<220>
<223> Probe 8 for determining the expression of the PARP3 gene

<400> 426
tctaccagga gagccagtgt cgcct 25

<210> 427
<211> 25
<212> DNA
<213> Artificial

<220>
<223> Probe 9 for determining the expression of the PARP3 gene

<400> 427
gggtcctgca aggctggact gtgat 25

<210> 428
<211> 25
<212> DNA
<213> Artificial

<220>
<223> Probe 10 for determining the expression of the PARP3 gene

<400> 428
gactgtgatc ttcaatcatc ctgcc 25

<210> 429
<211> 25
<212> DNA
<213> Artificial

<220>
<223> Probe 11 for determining the expression of the PARP3 gene

<400> 429
ccctatatca ctccttttt tcaag 25

<210> 430
<211> 25
<212> DNA
<213> Artificial

<220>
<223> Probe 1 for determining the expression of the CBX7 gene

<400> 430
ccccacccgc tttgaatgta gagac 25

<210> 431
<211> 25
<212> DNA
<213> Artificial

<220>
<223> Probe 2 for determining the expression of the CBX7 gene

<400> 431
tgtagagacc cgtgggcact tttcc 25

<210> 432
<211> 25
<212> DNA
<213> Artificial

<220>
<223> Probe 3 for determining the expression of the CBX7 gene

<400> 432
cacagccgcc tggaatgcag gactg 25

<210> 433
<211> 25
<212> DNA
<213> Artificial

<220>
<223> Probe 4 for determining the expression of the CBX7 gene

<400> 433
cactgctgtt cgggtgatga cctcg 25

<210> 434
<211> 25
<212> DNA
<213> Artificial

<220>
<223>  Probe 5 for determining the expression of the CBX7 gene

<400>  434
gctgtgcttc tgtgaggtgg tttag                                              25

<210>  435
<211>  25
<212>  DNA
<213>  Artificial

<220>
<223>  Probe 6 for determining the expression of the CBX7 gene

<400>  435
gctttcgaag tggccagctg cggcc                                              25

<210>  436
<211>  25
<212>  DNA
<213>  Artificial

<220>
<223>  Probe 7 for determining the expression of the CBX7 gene

<400>  436
ccaggtctca gcacaagagc gcttc                                              25

<210>  437
<211>  25
<212>  DNA
<213>  Artificial

<220>
<223>  Probe 8 for determining the expression of the CBX7 gene

<400>  437
agcgcttcct ttgcacagaa tgagc                                              25

<210>  438
<211>  25
<212>  DNA
<213>  Artificial

<220>
<223>  Probe 9 for determining the expression of the CBX7 gene

<400>  438
agcttcgagc tttgttcaga ctaaa                                              25

<210>  439
<211>  25
<212>  DNA

```
<213>  Artificial

<220>
<223>  Probe 10 for determining the expression of the CBX7 gene

<400>  439
gtcgggggca cgagttgatt ccaag                                              25


<210>  440
<211>  25
<212>  DNA
<213>  Artificial

<220>
<223>  Probe 11 for determining the expression of the CBX7 gene

<400>  440
gattccaagc acatgccttt gctga                                              25
```